# FASCICULE DE BREVET EUROPEEN

(11) **EP 3 814 532 B1**
(45) Date de publication et mention de la délivrance du brevet: **19.11.2025**
(21) Numéro de dépôt: 19733060.8
(22) Date de dépôt: 28.06.2019
(51) Int. Cl.: C12Q 1/6883

(54) **PROCÉDÉ POUR DÉTERMINER IN VITRO OU EX VIVO LE STATUT IMMUNITAIRE D'UN INDIVIDU**
VERFAHREN ZUR BESTIMMUNG DES IMMUNSTATUS EINES INDIVIDUUMS IN VITRO ODER EX VIVO
METHOD FOR DETERMINING IN VITRO OR EX VIVO THE IMMUNE STATUS OF AN INDIVIDUAL

(30) Priorité: 29.06.2018 EP 18180991; 12.09.2018 FR 1858167
(43) Date de publication de la demande: 05.05.2021
(73) Titulaire: bioMérieux, 69280 Marcy l'Etoile (FR)
(72) Inventeur: MOMMERT, Marine, 69003 Lyon (FR); TABONE, Olivier, 69007 Lyon (FR); TEXTORIS, Julien, 69100 Villeurbanne (FR); MALLET, François, 69100 Villeurbanne (FR)
(74) Mandataire: Germain Maureau
(86) Numéro de dépôt international: PCT/EP2019/067330
(87) Numéro de publication internationale: WO 2020/002602

(56) Documents cités:
- EP-A1- 2 565 270
- EP-A2- 2 186 913
- WO-A1-2012/101387
- WO-A2-2004/042346
- US-A1- 2016 319 352
- BECKER ET AL.: "A comprehensive hybridization model allows whole HERV transcriptome profiling using high density microarray", BMC GENOMICS, vol. 18, 286, 2017, pages 1 - 14, XP021243930

## Description

La présente invention a pour objet un procédé pour déterminer *in vitro* ou *ex vivo* le statut immunitaire d'un individu, de préférence un patient, comprenant une étape de détection et/ou de quantification de l'expression, dans un échantillon biologique dudit individu, d'une ou plusieurs séquences de HERV/MaLR, ainsi que les outils pour le mettre en œuvre et les utilisations de ceux-ci.

Le système immunitaire est un système de défense de l'organisme contre ce qui est reconnu comme du non-soi, tel que des pathogènes (e.g. virus, bactéries, parasites). Chez les mammifères, on distingue deux grands types de mécanismes : un mécanisme de défense non-spécifique, appelé également immunité « innée » ou « naturelle », et un mécanisme de défense spécifique, appelé également immunité « acquise » ou « adaptative ».

Ces réponses immunitaires nécessitent une régulation très fine. Chez un individu sain, la réponse immunitaire sera qualifiée de « normale » (on pourra également parler de statut immunitaire d'immunocompétence). Cependant, la réponse immunitaire peut parfois se retrouver altérée. On parlera de statut d'inflammation ou statut immunitaire hyperactif quand le système immunitaire est plus actif que la normale, comme dans le cas de maladies inflammatoires ou auto-immunes. Dans les maladies auto-immunes, le système immunitaire de l'organisme déclenche une réaction inflammatoire avec une immunisation caractérisée envers les antigènes du soi. A l'inverse, on parlera de statut d'immunosuppression (ou immunodépression ou immunodéficience ou statut immunitaire hypoactif ou paralysie immunitaire), quand le système immunitaire est moins actif que la normale.

L'immunosuppression peut avoir différentes origines, prendre de nombreuses formes, et affecter l'immunité innée et/ou l'immunité adaptative. En particulier, le sepsis est un problème de santé publique majeur, qui représente la première cause de mortalité en unité de soins intensifs. On estime que 28 millions d'individus développent un sepsis chaque année à travers le monde, parmi lesquels 8 millions décèderont de la pathologie (Fleischmann et al. (2016) American journal of respiratory and critical care medicine;193(3):259-72). Chez un patient atteint de sepsis (dit également, en état septique), la réponse immunitaire est dérégulée, suite à une infection, ce qui conduit à une défaillance et des dysfonctions d'organes multiples et potentiellement mortelles. Cette réponse immunitaire est complexe et évolue en fonction du temps, avec des phénomènes pro-inflammatoires et anti-inflammatoires excessifs et pouvant être concomitants. L'ensemble de ces dérèglements du système immunitaire conduit aux défaillances d'organes, à la paralysie du système immunitaire et aux infections secondaires. Le choc septique est un sous-type de sepsis, dans lequel une hypotension persiste, malgré un remplissage vasculaire adéquat (Singer et al (2016) JAMA; 315(8): 801-810). Au stade initial du sepsis, c'est une réponse inflammatoire, voire hyper-inflammatoire, qui semble prédominer, et qui est à l'origine de dommages tissulaires et de défaillances organiques, notamment au niveau rénal. C'est pourquoi les essais cliniques dans le domaine du sepsis se sont pendant longtemps concentrés sur des traitements anti-inflammatoires, mais avec des résultats peu concluants. Des études plus récentes sur la physiopathologie du sepsis ont conduit à montrer qu'une réponse anti-inflammatoire ou d'immunosuppression, survenait chez certains patients en état septique, soit de manière concomitante à l'inflammation initiale, soit plus tard. Le patient peut alors se retrouver dans un état d'immunosuppression, potentiellement sévère, en fonction des degrés respectifs des réponses pro-inflammatoires et anti-inflammatoires. Ces patients immunodéprimés présentent un risque élevé de développer des infections nosocomiales (ou HAI, *Health-care Associated Infections*)*,* et pourraient avantageusement bénéficier de traitements immunostimulants.

Il apparaît donc important de pouvoir déterminer le statut immunitaire d'un individu, et en particulier de pouvoir identifier un statut d'immunosuppression, afin de pouvoir adapter la prise en charge thérapeutique. Or, les individus ayant des dérèglements du système immunitaire ne présentent pas de signes cliniques spécifiques. Il existe donc un besoin important d'identification de biomarqueurs, permettant de déterminer le statut immunitaire d'un individu.

Actuellement, le test de référence pour suivre les altérations immunitaires chez les patients en soins intensifs (e.g. patients atteints de sepsis, de traumatismes, ayant subi une chirurgie lourde, brûlés, ou patients atteints de pancréatites) est la diminution de l'expression du HLA-DR (*human leucocyte antigen - D related*) *à* la surface des monocytes (mHLA-DR), mesurée par cytométrie en flux. En effet, ce marqueur fournit des informations précieuses en terme de prédiction de la mortalité ou encore de l'évaluation du risque d'infections secondaires chez ces patients. Le HLA-DR est un récepteur de surface appartenant au CMH (complexe majeur d'histocompatibilité) de classe Il. La mesure de l'expression du mHLA-DR représente en particulier le *gold standard* pour identifier si un patient atteint de sepsis est immunodéprimé ou non (Monneret et Venet (2016) Cytometry Part B (Clinical Cytometry) 90B:376-386). Cependant, cette approche nécessite d'importantes manipulations pré-analytiques de l'échantillon (Monneret et Venet (2014) Monocyte HLA-DR in sepsis: shall we stop following the flow? Crit Care 18:102). Par ailleurs, l'accessibilité à un cytomètre en flux n'est pas toujours possible dans tous les hôpitaux, et la mesure est difficile à standardiser d'un hôpital à un autre, voire d'un manipulateur à un autre.

Pour pallier ces inconvénients, d'autres biomarqueurs, utilisant des outils de biologie moléculaire, ont été proposés, comme par exemple un biomarqueur basé sur le ratio du niveau d'expression, au niveau ARNm, de CD74 au jour J3 (suivant l'admission du patient au sein d'une structure médicale) sur le niveau d'expression de CD74 au jour J1. Le CD74 représente la chaîne invariante y du HLA-DR. Il a été montré que le ratio d'expression CD74 J3/J1 est associé avec l'apparition d'infections secondaires acquises en soins intensifs (Peronnet et al (2017) Intensive Care Medicine ;43(7):1013-20). La demande de brevet WO2012/101387 décrit quant à elle un procédé de détermination du statut immunitaire d'un individu, à partir de l'analyse de l'expression d'au moins deux gènes choisis parmi plusieurs groupes de gènes. La demande de brevet WO2004/42346 propose un test pour évaluer le statut immunitaire chez un patient dans lequel le niveau d'expression d'un ou plusieurs gènes est mesuré.

Il a également été proposé, dans la demande de brevet WO2013/156627, une méthode de détermination du statut immunodéprimé ou non-immunodéprimé, à partir de la détermination de la charge en anellovirus, dans un échantillon biologique. Cependant, aucun de ces biomarqueurs n'est venu à ce jour remplacer l'utilisation du mHLA-DR. Ces biomarqueurs présentent notamment l'inconvénient de ne pas permettre d'identifier dans quelle phase se situe le patient (*i.e.* phase inflammatoire *versus* phase immunosuppressive), leur objectif étant essentiellement de pouvoir identifier les patients immunodéprimés, pour lesquels il serait pertinent d'administrer des traitements immunostimulants. Il a également été proposé, dans la publication « Becker et al. A comprehensive hybridization model allows whole HERV transcriptome profiling using high density microarray, BMC Genomics 2017 18:286 », l'utilisation d'une puce à ADN permettant l'identification de de séquences présentant un différentiel d'expression.

A la date de la présente invention, il demeure donc nécessaire de trouver de nouveaux biomarqueurs permettant de déterminer le statut immunitaire d'un individu.

Les rétrovirus endogènes, ou ERV (pour *Endogenous RetroVirus*) désignent des séquences stables du génome d'un organisme et ayant des analogies structurales avec certains rétrovirus exogènes infectieux (dont la présence de deux LTR, ou *Long Terminal Repeats,* qui encadrent les gènes codant pour les protéines putatives). Leur origine est incertaine, mais l'hypothèse la plus probable est celle de l'infection de cellules germinales par un rétrovirus. Suite à des mutations du rétrovirus qui l'auraient rendu défectif, les cellules germinales infectées auraient pu survivre, et le génome du rétrovirus, intégré dans le génome de l'organisme, aurait pu être transmis à la génération suivante, et persister dans la descendance au sein du génome de l'organisme.

Chez l'homme, les HERV (*Human Endogenous RetroVirus*) n'ont été mis en évidence que depuis le séquençage du génome humain. Avec les MaLR (*Mammalian apparent LTR-Retrotransposons*)*,* qui possèdent une structure similaire aux HERV, ils représentent 8,3% du génome humain, avec un nombre s'élevant à plus de 400 000 éléments. A titre de comparaison, les 30 000 à 40 000 gènes codant pour des protéines, représentent uniquement 2% de l'ADN humain. Les HERV sont subdivisés en trois grandes classes (I, Il et III) et plusieurs groupes (parfois appelés « familles », dans la présente demande de brevet). Les HERV sont des rétroéléments qui se transposent uniquement par un mode de copier-coller, par le biais d'un intermédiaire à ARN et d'une transcriptase inverse. Ils ont longtemps été considérés comme de l'ADN « poubelle » (*junk DNA*)*.* S'ils peuvent être inactifs à cause de mutations ou par des mécanismes épigénétiques, leur rôle commence à apparaître, aussi bien dans des contextes physiologiques que pathologiques. Ainsi, il a été montré que HERV-W participe à un des mécanismes assurant la formation du placenta. La superfamille HERV-K est quant à elle la plus étudiée en relation avec la carcinogenèse. L'expression de certains HERV a également été décrite dans certaines maladies auto-immunes, comme la sclérose en plaques ou le lupus érythémateux, et dans les interféronopathies, sans que rien n'évoque un possible lien entre réactivation des HERV et le statut immunitaire.

Ainsi, il n'a jamais été décrit ni suggéré que l'analyse de l'expression de HERV chez l'Homme pouvait permettre de déterminer le statut immunitaire d'un individu. Par ailleurs, la réactivation des HERV n'a jamais été décrite dans la pathologie du sepsis.

Or, il a été découvert que, de façon tout-à-fait surprenante, parmi les quelque 420 000 HERV/MaLR existants, l'analyse de l'expression de certains d'entre eux permettait de déterminer le statut immunitaire d'un individu.

Ainsi, la présente invention a pour objet un procédé pour déterminer *in vitro* ou *ex vivo* le statut immunitaire d'un individu, dans lequel l'individu est un patient atteint de traumatismes, un patient atteint de brûlures, un patient ayant reçu une chirurgie ou un patient en état septique, de préférence un patient atteint de choc septique, ledit procédé comprenant une étape de détection et/ou de quantification de l'expression, dans un échantillon biologique test dudit individu d'au moins une partie, de préférence d'une taille d'au moins 10, au moins 11, au moins 12, au moins 13, au moins 14, au moins 15, au moins 16, au moins 17, au moins 18, au moins 19, au moins 20, au moins 21, au moins 22, au moins 23, au moins 24, au moins 25, au moins 26, au moins 27, au moins 28, au moins 29, au moins 30 nucléotides, d'au moins une séquence de HERV/MaLR choisie parmi les séquences identifiées en SEQ ID NOs : 1 à 34 ou parmi les séquences qui présentent au moins 99%, de préférence au moins 99,1%, de préférence au moins 99,2%, de préférence au moins 99,3%, de préférence au moins 99.4%, de préférence au moins 99.5%, de préférence au moins 99.6%, de préférence au moins 99.7%, de préférence au moins 99.8%, de préférence au moins 99.9% d'identité avec une des séquences identifiées en SEQ ID NOs : 1 à 34, parmi les listes suivantes :

**Tableau 1. Liste 1**

| **SEQ ID NO** | **Localisation GRCh38** | **Nom du probeset correspondant de la puce HERV-V3** | **Nom du groupe de HERV/MaLR** |
|---|---|---|---|
| 1 | chr19:54891074-54891496 | 190665001-HERV0376 | HERV0376 |
| 2 | chr22:36153696-36154283 | 220247002-HERV0797 | HERV0797 |
| 3 | chr17:35505737-35508365 | 170369402HE41env | HERV-E41 |
| 4 | chr12:112971073-112971451 | 121601801-HERV0492 | HERV0492 |
| 5 | chr1:78648318-78648697 | 011052702-MALR1044 | MALR1044 |
| 6 | chr1:78623489-78623954 | 011052202-HERV1033 | HERV1033 |
| 7 | chr13:42884951-42886257 | 130360601-HERV0808 | HERV0808 |
| 8 | chr14:91230494-91230820 | 141107102-MALR1019 | MALR1019 |
| 9 | chr2:102363654-102366601 | 021460102-HERV0599uL | HERV0599 |
| 10 | chr2:102013616-102013971 | 021456001-MALR1017uL | MALR1017 |
| 11 | chr5:14551189-14551685 | 050286701-HERV0513 | HERV0513 |
| 12 | chr5:14562791-14563322 | 050287402-MALR1022 | MALR1022 |

**Tableau 2. Liste 2**

| **SEQ ID NO** | **Localisation GRCh38** | **Nom du probeset correspondant de la puce HERV-V3** | **Nom du groupe de HERV/MaLR** |
|---|---|---|---|
| 13 | chr5:132453630-132454148 | 052182701-MALR1129 | MALR1129 |
| 14 | chr19:41812466-41813010 | 190478501-MALR1003 | MALR1003 |
| 15 | chr1:155637287-155637547 | 011790601ERV9sLU5 | ERV9 |
| 16 | chr5:170290289-170290812 | 052681601-MALR1018 | MALR1018 |
| 17 | chr16:50662453-50662912 | 160627301-MALR1014 | MALR1014 |
| 18 | chr11:122671887-122672147 | 111686702-HERV0861 | HERV0861 |
| 8 | chr14:91230494-91230820 | 141107102-MALR1019 | MALR1019 |
| 19 | chr4:15825146-15825565 | 040318302-MALR1134 | MALR1134 |
| 20 | chr4:83464568-83464963 | 041529101-MALR1026 | MALR1026 |
| 21 | chr14:91222760-91223118 | 141106902-MALR1133 | MALR1133 |

**Tableau 3. Liste 3**

| **SEQ ID NO** | **Localisation GRCh38** | **Nom du probeset correspondant de la puce HERV-V3** | **Nom du groupe de HERV/MaLR** |
|---|---|---|---|
| 1 | chr19:54891074-54891496 | 190665001-HERV0376 | HERV0376 |
| 1 | chr19:54891074-54891496 | 190665002-HERV0376 | HERV0376 |
| 22 | chr6:18403673-18404108 | 060281701-MALR1043 | MALR1043 |
| 23 | chr4:184850413-184850785 | 043166601-MALR1018 | MALR1018 |
| 24 | chr10:5856198-5856795 | 100090601-HERV0429 | HERV0429 |
| 25 | chr6:107800650-107801138 | 061529601-HERV0492 | HERV0492 |
| 26 | chr10:60410534-60411224 | 100871501-MALR1020 | MALR1020 |
| 27 | chr17:78345106-78345577 | 170842002-MALR1003 | MALR1003 |

**Tableau 4. Liste 4**

| **SEQ ID NO** | **Localisation GRCh38** | **Nom du probeset correspondant de la puce HERV-V3** | **Nom du groupe de HERV/MaLR** |
|---|---|---|---|
| 28 | chr8:125945973-125951030 | 081921103-HERV0958 | HERV0958 |
| 29 | chr3:167401329-167401866 | 032622601MR41sLU5p | MR41 |
| 30 | chr22:36147793-36148208 | 220246901-HERV0889 | HERV0889 |
| 31 | chr6:127790579-127792191 | 061827101-HERV0856 | HERV0856 |
| 3 | chr17:35505737-35508365 | 170369402HE41env | HERV-E41 |
| 32 | chr17:77462942-77463350 | 170828901-HERV0770 | HERV0770 |
| 28 | chr8:125945973-125951030 | 081921101-HERV0958 | HERV0958 |
| 28 | chr8:125945973-125951030 | 081921102-HERV0958 | HERV0958 |
| 33 | chr19:14612123-14612747 | 190148802-MALR1127 | MALR1127 |
| 34 | chr12:9038254-9038598 | 120093401-HERV1034 | HERV1034 |

Dans le cadre de la présente invention :
- « déterminer le statut immunitaire » d'un individu consiste à évaluer la capacité de l'organisme à mettre en œuvre une réponse immunitaire et à se défendre contre des agressions ou des infections. Le statut immunitaire peut notamment être déterminé comme étant un statut immunitaire normal (ou statut d'immunocompétence), un statut d'inflammation (ou statut immunitaire hyperactif) quand le système immunitaire est plus actif que la normale, ou un statut d'immunosuppression (ou immunodépression ou immunodéficience ou statut immunitaire hypoactif ou paralysie immunitaire), quand le système immunitaire est moins actif que la normale.
- Par « HERV/MaLR », on entend les éléments de type HERV et MaLR, tels que présentés en introduction. Le terme abrégé de « HERV » pourra également parfois être utilisé, et avoir la même signification que « HERV/MaLR ». Dans la littérature, de nombreux alias ont été utilisés pour décrire un même élément HERV ou un même groupe (ou famille) de HERV, et il existe encore à ce jour un besoin de standardisation. Dans la présente demande, pour éviter toute confusion, on se réfèrera en premier lieu, pour déterminer l'identité d'un élément HERV, à la localisation chromosomique de l'élément HERV en question, plus particulièrement en se basant sur le GRCh38 (*Genome Reference Consortium Human Build 38*)*.* On pourra également parfois se référer aux différents probesets et aux différentes sondes de la puce HERV-V3 ciblant l'élément HERV en question. Il convient également de noter que, pour un élément HERV donné, tel qu'identifié par sa localisation GRCh38, les séquences retrouvées chez différents individus peuvent différer d'avec la séquence indiquée dans la base GRCh38, du fait du polymorphisme (Wildschutte et al (2016), Discovery of unfixed endogenous retrovirus insertions in diverse human populations, PNAS 113(16):E2326-34).
- Le terme « individu » désigne un être humain, quel que soit son état de santé. Un « individu sain » au sens de la présente invention est un individu qui ne présente pas de dérèglement du système immunitaire. Le terme « patient » désigne un individu qui est entré en contact avec un professionnel de la santé, tel qu'un médecin (par exemple, un médecin généraliste) ou une structure médicale (par exemple, le service des urgences ou de réanimation d'un hôpital, ou une unité de soins intensifs)
- Par « détection de l'expression d'une séquence », on entend la mise en évidence de l'expression de ladite séquence, sans nécessairement une mesure quantitative. Dans le cas d'un transcrit ARNm, la détection peut être réalisée par une méthode directe, par tout procédé connu de l'homme du métier permettant de déterminer la présence dudit transcrit dans un échantillon, ou par détection indirecte du transcrit après transformation de ce dernier en ADN, ou après amplification dudit transcrit ou après amplification de l'ADN obtenu après transformation dudit transcrit en ADN. La « quantification de l'expression d'une séquence » désigne le fait d'évaluer le niveau d'expression de la séquence, de manière quantitative. De nombreuses méthodes existent pour la détection des acides nucléiques (voir par exemple Kricka et al., Clinical Chemistry, 1999, n° 45(4), p.453-458 ; Relier G. H. et al., DNA Probes, 2nd Ed., Stockton Press, 1993, sections 5 et 6, p.173-249).
- Par « échantillon biologique », on se réfère à tout échantillon provenant d'un individu, et pouvant être de différentes natures, comme le sang, le sérum, le plasma, les expectorations, l'urine, les selles, la peau, le liquide céphalo-rachidien, le liquide de lavage broncho-alvéolaire, la salive, les sécrétions gastriques, le sperme, le liquide séminal, les larmes, la moelle épinière, ganglion du nerf trijumeau, tissu adipeux, tissu lymphoïde, tissu placentaire, tissu du tractus gastrointestinal, tissu du tractus génital, tissu du système nerveux central. En particulier, cet échantillon peut être un fluide biologique, de préférence choisi parmi le sang total (tel que collecté de la voie veineuse, c'est-à-dire contenant les cellules blanches et rouges, les plaquettes et le plasma), le plasma et le sérum. Il peut également s'agir de tout type de cellules extraites à partir d'un échantillon de sang, telles que les cellules mononucléées sanguines périphériques (ou PBMC), des sous-populations de cellules B, des monocytes purifiés, ou des neutrophiles.
- Afin de déterminer le « pourcentage d'identité de séquence » d'une séquence d'acides nucléiques avec une autre séquence d'acides nucléiques, les deux séquences sont d'abord alignées de manière optimale. Les deux séquences à comparer peuvent être de même taille ou de tailles différentes. Dans certains cas, il peut être nécessaire d'introduire des « trous » dans l'une des séquences, afin de permettre un alignement optimal avec la seconde séquence. L'alignement optimal des séquences peut notamment être réalisé en utilisant l'algorithme de Smith et Waterman (J. Theor. Biol., 91 (2): 370-380, 1981 ), l'algorithme de Needleman and Wunsch (J. Mol. Biol, 48(3) : 443-453, 1972), ou la méthode de Pearson and Lipman (Proc. Natl. Acad. Sri. U. S.A. , 85(5) : 2444-2448, 1988). Certains logiciels permettent d'implémenter certains de ces algorithmes, tels que GAP, BESTFIT, FASTA, TFASTA (Wisconsin Genetics Software Package Release 7.0, Genetic Computer Group, 575, Science Drive, Madison, Wisconsin), BLAST ou encore CLUSTALW (Nucleic Acids Res. 1994 Nov 11; 22(22) :4673-80. *CLUSTAL W: improving the sensitivity of progressive multiple sequence alignment through sequence weighting, position-specific gap penalties and weight matrix choice*)*.* Le meilleur alignement (*i.e.* celui permettant d'obtenir le pourcentage d'identité le plus élevé sur la fenêtre de comparaison), parmi ceux générés par ces diverses méthodes, est sélectionné. On compare ensuite les nucléotides respectifs se situant à la même position de chacune des séquences. Quand une position donnée est occupée par le même nucléotide dans les deux séquences, alors les séquences sont identiques pour cette position. Le pourcentage d'identité de séquence est alors déterminé en fonction du nombre de positions pour lesquelles les nucléotides respectifs sont identiques, rapporté au nombre total de nucléotides des positions pour lesquelles l'alignement a été possible, au niveau de la fenêtre de comparaison.
- Un « biomarqueur » ou « marqueur » est une caractéristique biologique mesurable objectivement qui représente un indicateur des processus biologiques normaux ou pathologiques ou de réponse pharmacologique à une intervention thérapeutique. Il peut s'agir en particulier d'un biomarqueur moléculaire, de préférence détectable au niveau ARNm. Plus particulièrement, le biomarqueur peut être un biomarqueur endogène ou loci (tel qu'un HERV ou un gène, qui se retrouvent dans le matériel chromosomique d'un individu) ou un biomarqueur exogène (tel qu'un virus).
- Le « sepsis » est une pathologie dans laquelle la réponse immunitaire est dérégulée chez un individu, suite à une infection, conduisant à une défaillance et des dysfonctions d'organes multiples et potentiellement mortelles. Le « choc septique » est un sous-type de sepsis, dans lequel une hypotension persiste, malgré un remplissage vasculaire adéquat.
- On entend par « amorce d'amplification », un fragment nucléotidique pouvant comprendre de 5 à 100 nucléotides, de préférence de 15 à 30 nucléotides, et possédant une spécificité d'hybridation avec une séquence nucléotidique cible, dans des conditions déterminées pour l'initiation d'une polymérisation enzymatique, par exemple dans une réaction d'amplification enzymatique de la séquence nucléotidique cible. Généralement, on utilise des « couples d'amorces », constitués de deux amorces. Lorsque l'on souhaite réaliser l'amplication de plusieurs HERV différents, plusieurs couples d'amorces différents sont de préférence utilisés, ayant préférentiellement chacun une capacité à s'hybrider spécifiquement avec un HERV différent.
- On entend par « sonde d'hybridation », un fragment nucléotidique comprenant typiquement de 5 à 100 nucléotides, de préférence de 15 à 90 nucléotides, de manière encore plus préférée de 15 à 35 nucléotides, possédant une spécificité d'hybridation dans des conditions déterminées pour former un complexe d'hybridation avec une séquence nucléotidique cible. La sonde comporte également un rapporteur (tel qu'un fluorophore, une enzyme ou tout autre système de détection), qui va permettre la détection de la séquence nucléotidique cible. Dans la présente invention, la séquence nucléotidique cible peut être une séquence nucléotidique comprise dans un ARN messager (ARNm) ou une séquence nucléotidique comprise dans un ADN complémentaire (ADNc) obtenu par transcription inverse dudit ARNm. Lorsque l'on souhaite cibler plusieurs HERV différents, plusieurs sondes différentes sont de préférence utilisées, ayant préférentiellement chacune une capacité à s'hybrider spécifiquement avec un HERV différent.
- Par « hybridation », on entend le processus au cours duquel, dans des conditions appropriées, deux fragments nucléotidiques, tels que par exemple une sonde d'hybridation et un fragment nucléotidique cible, ayant des séquences suffisamment complémentaires, sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier. En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.
- Par « réaction d'amplification enzymatique », on entend un processus générant de multiples copies d'un fragment nucléotidique cible, par l'action d'au moins une enzyme. De telles réactions d'amplification sont bien connues de l'homme du métier et on peut citer notamment les techniques suivantes : PCR (*Polymerase Chain Reaction*) *,* LCR (*Ligase Chain Reaction*)*,* RCR (*Repair Chain Reaction*)*,* 3SR (*Self Sustained Séquence Replication*) avec la demande de brevet WO-A-90/06995, NASBA (*Nucleic Acid Sequence-Based Amplification*)*,* TMA (*Transcription Mediated Amplification*) avec le brevet US-A-5,399,491, et LAMP (*Loop mediated isothermal amplification*) avec le brevet US6410278. Lorsque la réaction d'amplification enzymatique est une PCR, on parlera plus particulièrement de RT-PCR (RT pour « *reverse transcription* »)*,* lorsque l'étape d'amplification est précédée d'une étape de réverse-transcription d'ARN messager (ARNm) en ADN complémentaire (ADNc), et de qPCR ou RT-qPCR lorsque la PCR est quantitative.

A la connaissance des inventeurs, il n'a jamais été décrit ni suggéré que la détection et/ou la quantification de l'expression de HERV/MaLR pouvait permettre de déterminer le statut immunitaire d'un individu. En particulier, l'implication des HERV/MaLR n'a jamais été décrite dans le domaine du sepsis.

L'ensemble des HERV/MaLR de SEQ ID NO :1 à 34 ont été identifiés parmi les quelque 420 000 HERV/MaLR du génome. Il s'agit de HERV/MaLR qu'on arrive à cibler avec la puce HERV-V3 (Becker et al, BMC Genomics. 2017; 18: 286). Plus particulièrement, ces HERV/MaLR sont exprimés dans les jeux de données utilisés dans les Exemples. Encore plus particulièrement, ces HERV/MaLR exprimés sont modulés entre les conditions d'intérêt retenues dans les Exemples.

La détection et/ou la quantification de l'expression des HERV selon la présente invention, pour déterminer le statut immunitaire d'un individu, peut s'effectuer au moyen d'outils moléculaires qui présentent des avantages par rapport à l'utilisation de la cytométrie en flux pour la mesure de mHLA-DR, en termes d'accessibilité au sein des hôpitaux et de standardisation. Peu de manipulation des échantillons est nécessaire et les résultats sont faciles à interpréter. Par ailleurs, l'expression des HERV selon la présente invention peut être détectée et/ou quantifiée sur plusieurs types de plateformes, comme des puces à ADN ou par PCR, afin de déterminer le statut immunitaire. Certains des HERV selon la présente invention peuvent être détectés précocement, dès J1, alors que la mesure de mHLA-DR s'effectue à J3 (Monneret et Venet (2014) Monocyte HLA-DR in sepsis: shall we stop following the flow? Crit Care 18:102).

Préférentiellement, la présente invention a pour objet un procédé pour déterminer in *vitro* ou *ex vivo* le statut immunitaire d'un individu, dans lequel l'individu est un patient atteint de traumatismes, un patient atteint de brûlures, un patient ayant reçu une chirurgie ou un patient en état septique, de préférence un patient atteint de choc septique, ledit procédé comprenant :
- une étape de détection et/ou de quantification de l'expression, dans un échantillon biologique dudit individu (ou échantillon biologique test), d'au moins une partie d'au moins une séquence de HERV/MaLR choisie parmi les séquences identifiées en SEQ ID NOs : 1 à 34 ou parmi les séquences qui présentent au moins 99% d'identité avec une des séquences identifiées en SEQ ID NOs : 1 à 34, parmi les Listes 1 à 4, précédemment décrites ;
- une étape dans laquelle on compare l'expression dans l'échantillon biologique test, avec une expression de référence, ou avec l'expression dans un échantillon biologique de référence ;
- une étape dans laquelle on détermine le statut immunitaire de l'individu à partir de cette comparaison.

L'échantillon biologique de référence peut être de différentes natures, mais il est de préférence de nature identique, ou au moins d'une nature proche, à la nature de l'échantillon biologique test. Par exemple, si l'échantillon biologique test est un échantillon de sang total, l'échantillon biologique de référence sera de préférence un échantillon de sang total, ou éventuellement un échantillon de plasma ou de sérum. L'échantillon biologique de référence peut être une échantillon « naturel », c'est-à-dire provenant d'un individu dont le statut immunitaire est connu ou déterminé selon une méthode de référence (par exemple, par la méthode mHLA-DR). Il peut par exemple provenir d'un individu de statut immunitaire connu comme étant un statut d'immunocompétence, un statut d'inflammation, ou un statut d'immunosuppression. De préférence, si l'échantillon biologique test provient d'un être humain, l'échantillon biologique de référence provient également d'un être humain. De manière encore plus préférée, l'échantillon biologique de référence provient du même individu que celui dont provient l'échantillon biologique test. L'échantillon biologique de référence peut également être un échantillon « synthétique », c'est-à-dire un échantillon contenant une quantité calibrée d'au moins une des séquences SEQ ID NO :1 à 34.

L'invention a préférentiellement pour objet un procédé pour déterminer le statut immunitaire d'un individu, tel que décrit précédemment, dans lequel on détecte et/ou on quantifie l'expression d'au moins 2 séquences différentes choisies parmi les séquences identifiées en SEQ ID NOs : 1 à 34 ou parmi les séquences qui présentent au moins 99% d'identité avec une des séquences identifiées en SEQ ID NOs : 1 à 34. Les Listes 1 à 4 étant complémentaires entre elles, ces au moins deux séquences différentes sont préférentiellement choisies dans deux listes différentes.

L'invention a préférentiellement pour objet un procédé pour déterminer le statut immunitaire d'un individu, tel que décrit précédemment, dans lequel on détecte et/ou on quantifie l'expression d'au moins 3 séquences différentes choisies parmi les séquences identifiées en SEQ ID NOs : 1 à 34 ou parmi les séquences qui présentent au moins 99% d'identité avec une des séquences identifiées en SEQ ID NOs : 1 à 34. Ces au moins trois séquences différentes sont préférentiellement choisies dans deux listes différentes, de préférence encore dans trois listes différentes.

L'invention a préférentiellement pour objet un procédé pour déterminer le statut immunitaire d'un individu, tel que décrit précédemment, dans lequel on détecte et/ou on quantifie l'expression d'au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 21, au moins 22, au moins 23, au moins 24, au moins 25, au moins 26, au moins 27, au moins 28, au moins 29, au moins 30, au moins 31, au moins 32, au moins 33, au moins 34 séquences différentes choisies parmi les séquences identifiées en SEQ ID NOs : 1 à 34 ou parmi les séquences qui présentent au moins 99% d'identité avec une des séquences identifiées en SEQ ID NOs : 1 à 34. Ces au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 21, au moins 22, au moins 23, au moins 24, au moins 25, au moins 26, au moins 27, au moins 28, au moins 29, au moins 30, au moins 31, au moins 32, au moins 33, au moins 34 séquences différentes sont préférentiellement choisies dans deux listes différentes, de préférence encore dans trois listes différentes, de manière encore plus préférée dans quatre listes différentes.

Les HERV de SEQ ID NO : 1, 3 et 8 ont été identifiés à partir de deux stratégies différentes, tel que décrit dans les Exemples, et se retrouvent ainsi respectivement dans deux Listes. Par ailleurs, les inventeurs ont attribué une note pour les différentes séquences de HERV, comme expliqué dans les Exemples 4 à 6. Dans l'Exemple 7, les HERV ont été classés par ordre d'importance. Aussi, de manière tout-à-fait préférée, l'invention a pour objet un procédé pour déterminer le statut immunitaire d'un individu, tel que décrit précédemment, dans lequel on détecte et/ou on quantifie l'expression d'au moins 1, au moins 2, au moins 3, au moins 4, au moins 5, au moins 6, au moins 7 séquences différentes choisies parmi les séquences identifiées en SEQ ID NO : 1, SEQ ID NO : 3, SEQ ID NO : 8, SEQ ID NO : 11, SEQ ID NO : 12, SEQ ID NO : 13 et SEQ ID NO : 28, ou parmi les séquences qui présentent au moins 99%, au moins 99,1%, au moins 99,2%, au moins 99,3%, au moins 99.4%, au moins 99.5%, au moins 99.6%, au moins 99.7%, au moins 99.8%, au moins 99.9% d'identité avec une des séquences SEQ ID NO : 1, 3, 8, 11, 12, 13 et 28. Les combinaisons préférées d'au moins 2 HERV correspondantes sont listées dans le Tableau 5 ci-dessous.

**Tableau 5**

| **Nombre de HERV dans la combinaison** | **Combinaisons préférées d'au moins 2 HERV** |
|---|---|
| 2 | SEQ ID NO : 1,SEQ ID NO : 3 |
| | SEQ ID NO : 1,SEQ ID NO : 8 |
| | SEQ ID NO : 1,SEQ ID NO : 11 |
| | SEQ ID NO : 1,SEQ ID NO : 12 |
| | SEQ ID NO : 1,SEQ ID NO : 13 |
| | SEQ ID NO : 1,SEQ ID NO : 28 |
| | SEQ ID NO : 3,SEQ ID NO : 8 |
| | SEQ ID NO : 3,SEQ ID NO : 11 |
| | SEQ ID NO : 3,SEQ ID NO : 12 |
| | SEQ ID NO : 3,SEQ ID NO : 13 |
| | SEQ ID NO : 3,SEQ ID NO : 28 |
| | SEQ ID NO : 8,SEQ ID NO : 11 |
| | SEQ ID NO : 8,SEQ ID NO : 12 |
| | SEQ ID NO : 8,SEQ ID NO : 13 |
| | SEQ ID NO : 8,SEQ ID NO : 28 |
| | SEQ ID NO : 11,SEQ ID NO : 12 |
| | SEQ ID NO : 11,SEQ ID NO : 13 |
| | SEQ ID NO : 11,SEQ ID NO : 28 |
| | SEQ ID NO : 12,SEQ ID NO : 13 |
| | SEQ ID NO : 12,SEQ ID NO : 28 |
| | SEQ ID NO : 13,SEQ ID NO : 28 |
| 3 | SEQ ID NO : 1,SEQ ID NO : 3,SEQ ID NO : 8 |
| | SEQ ID NO : 1,SEQ ID NO : 3,SEQ ID NO : 11 |
| | SEQ ID NO : 1,SEQ ID NO : 3,SEQ ID NO : 12 |
| | SEQ ID NO : 1,SEQ ID NO : 3,SEQ ID NO : 13 |
| | SEQ ID NO : 1,SEQ ID NO : 3,SEQ ID NO : 28 |
| | SEQ ID NO : 1,SEQ ID NO : 8,SEQ ID NO : 11 |
| | SEQ ID NO : 1,SEQ ID NO : 8,SEQ ID NO : 12 |
| | SEQ ID NO : 1,SEQ ID NO : 8,SEQ ID NO : 13 |
| | SEQ ID NO : 1,SEQ ID NO : 8,SEQ ID NO : 28 |
| | SEQ ID NO : 1,SEQ ID NO : 11,SEQ ID NO : 12 |
| | SEQ ID NO : 1,SEQ ID NO : 11,SEQ ID NO : 13 |
| | SEQ ID NO : 1,SEQ ID NO : 11,SEQ ID NO : 28 |
| | SEQ ID NO : 1,SEQ ID NO : 12,SEQ ID NO : 13 |
| | SEQ ID NO : 1,SEQ ID NO : 12,SEQ ID NO : 28 |
| | SEQ ID NO : 1,SEQ ID NO : 13,SEQ ID NO : 28 |
| | SEQ ID NO : 3,SEQ ID NO : 8,SEQ ID NO : 11 |
| | SEQ ID NO : 3,SEQ ID NO : 8,SEQ ID NO : 12 |
| | SEQ ID NO : 3,SEQ ID NO : 8,SEQ ID NO : 13 |
| | SEQ ID NO : 3,SEQ ID NO : 8,SEQ ID NO : 28 |
| | SEQ ID NO : 3,SEQ ID NO : 11,SEQ ID NO : 12 |
| | SEQ ID NO : 3,SEQ ID NO : 11,SEQ ID NO : 13 |
| | SEQ ID NO : 3,SEQ ID NO : 11,SEQ ID NO : 28 |
| | SEQ ID NO : 3,SEQ ID NO : 12,SEQ ID NO : 13 |
| | SEQ ID NO : 3,SEQ ID NO : 12,SEQ ID NO : 28 |
| | SEQ ID NO : 3,SEQ ID NO : 13,SEQ ID NO : 28 |
| | SEQ ID NO : 8,SEQ ID NO : 11,SEQ ID NO : 12 |
| | SEQ ID NO : 8,SEQ ID NO : 11,SEQ ID NO : 13 |
| | SEQ ID NO : 8,SEQ ID NO : 11,SEQ ID NO : 28 |
| | SEQ ID NO : 8,SEQ ID NO : 12,SEQ ID NO : 13 |
| | SEQ ID NO : 8,SEQ ID NO : 12,SEQ ID NO : 28 |
| | SEQ ID NO : 8,SEQ ID NO : 13,SEQ ID NO : 28 |
| | SEQ ID NO : 11,SEQ ID NO : 12,SEQ ID NO : 13 |
| | SEQ ID NO : 11,SEQ ID NO : 12,SEQ ID NO : 28 |
| | SEQ ID NO : 11,SEQ ID NO : 13,SEQ ID NO : 28 |
| | SEQ ID NO : 12,SEQ ID NO : 13,SEQ ID NO : 28 |
| 4 | SEQ ID NO : 1,SEQ ID NO : 3,SEQ ID NO : 8,SEQ ID NO : 11 |
| | SEQ ID NO : 1,SEQ ID NO : 3,SEQ ID NO : 8,SEQ ID NO : 12 |
| | SEQ ID NO : 1,SEQ ID NO : 3,SEQ ID NO : 8,SEQ ID NO : 13 |
| | SEQ ID NO : 1,SEQ ID NO : 3,SEQ ID NO : 8,SEQ ID NO : 28 |
| | SEQ ID NO : 1,SEQ ID NO : 3,SEQ ID NO : 11,SEQ ID NO : 12 |
| | SEQ ID NO : 1,SEQ ID NO : 3,SEQ ID NO : 11,SEQ ID NO : 13 |
| | SEQ ID NO : 1,SEQ ID NO : 3,SEQ ID NO : 11,SEQ ID NO : 28 |
| | SEQ ID NO : 1,SEQ ID NO : 3,SEQ ID NO : 12,SEQ ID NO : 13 |
| | SEQ ID NO : 1,SEQ ID NO : 3,SEQ ID NO : 12,SEQ ID NO : 28 |
| | SEQ ID NO : 1,SEQ ID NO : 3,SEQ ID NO : 13,SEQ ID NO : 28 |
| | SEQ ID NO : 1,SEQ ID NO : 8,SEQ ID NO : 11,SEQ ID NO : 12 |
| | SEQ ID NO : 1,SEQ ID NO : 8,SEQ ID NO : 11,SEQ ID NO : 13 |
| | SEQ ID NO : 1,SEQ ID NO : 8,SEQ ID NO : 11,SEQ ID NO : 28 |
| | SEQ ID NO : 1,SEQ ID NO : 8,SEQ ID NO : 12,SEQ ID NO : 13 |
| | SEQ ID NO : 1,SEQ ID NO : 8,SEQ ID NO : 12,SEQ ID NO : 28 |
| | SEQ ID NO : 1,SEQ ID NO : 8,SEQ ID NO : 13,SEQ ID NO : 28 |
| | SEQ ID NO : 1,SEQ ID NO : 11,SEQ ID NO : 12,SEQ ID NO : 13 |
| | SEQ ID NO : 1,SEQ ID NO : 11,SEQ ID NO : 12,SEQ ID NO : 28 |
| | SEQ ID NO : 1,SEQ ID NO : 11,SEQ ID NO : 13,SEQ ID NO : 28 |
| | SEQ ID NO : 1,SEQ ID NO : 12,SEQ ID NO : 13,SEQ ID NO : 28 |
| | SEQ ID NO : 3,SEQ ID NO : 8,SEQ ID NO : 11,SEQ ID NO : 12 |
| | SEQ ID NO : 3,SEQ ID NO : 8,SEQ ID NO : 11,SEQ ID NO : 13 |
| | SEQ ID NO : 3,SEQ ID NO : 8,SEQ ID NO : 11,SEQ ID NO : 28 |
| | SEQ ID NO : 3,SEQ ID NO : 8,SEQ ID NO : 12,SEQ ID NO : 13 |
| | SEQ ID NO : 3,SEQ ID NO : 8,SEQ ID NO : 12,SEQ ID NO : 28 |
| | SEQ ID NO : 3,SEQ ID NO : 8,SEQ ID NO : 13,SEQ ID NO : 28 |
| | SEQ ID NO : 3,SEQ ID NO : 11,SEQ ID NO : 12,SEQ ID NO : 13 |
| | SEQ ID NO : 3,SEQ ID NO : 11,SEQ ID NO : 12,SEQ ID NO : 28 |
| | SEQ ID NO : 3,SEQ ID NO : 11,SEQ ID NO : 13,SEQ ID NO : 28 |
| | SEQ ID NO : 3,SEQ ID NO : 12,SEQ ID NO : 13,SEQ ID NO : 28 |
| | SEQ ID NO : 8,SEQ ID NO : 11,SEQ ID NO : 12,SEQ ID NO : 13 |
| | SEQ ID NO : 8,SEQ ID NO : 11,SEQ ID NO : 12,SEQ ID NO : 28 |
| | SEQ ID NO : 8,SEQ ID NO : 11,SEQ ID NO : 13,SEQ ID NO : 28 |
| | SEQ ID NO : 8,SEQ ID NO : 12,SEQ ID NO : 13,SEQ ID NO : 28 |
| | SEQ ID NO : 11,SEQ ID NO : 12,SEQ ID NO : 13,SEQ ID NO : 28 |
| 5 | SEQ ID NO : 1,SEQ ID NO : 3,SEQ ID NO : 8,SEQ ID NO : 11,SEQ ID NO : 12 |
| | SEQ ID NO : 1,SEQ ID NO : 3,SEQ ID NO : 8,SEQ ID NO : 11,SEQ ID NO : 13 |
| | SEQ ID NO : 1,SEQ ID NO : 3,SEQ ID NO : 8,SEQ ID NO : 11,SEQ ID NO : 28 |
| | SEQ ID NO : 1,SEQ ID NO : 3,SEQ ID NO : 8,SEQ ID NO : 12,SEQ ID NO : 13 |
| | SEQ ID NO : 1,SEQ ID NO : 3,SEQ ID NO : 8,SEQ ID NO : 12,SEQ ID NO : 28 |
| | SEQ ID NO : 1,SEQ ID NO : 3,SEQ ID NO : 8,SEQ ID NO : 13,SEQ ID NO : 28 |
| | SEQ ID NO : 1,SEQ ID NO : 3,SEQ ID NO : 11,SEQ ID NO : 12,SEQ ID NO : 13 |
| | SEQ ID NO : 1,SEQ ID NO : 3,SEQ ID NO : 11,SEQ ID NO : 12,SEQ ID NO : 28 |
| | SEQ ID NO : 1,SEQ ID NO : 3,SEQ ID NO : 11,SEQ ID NO : 13,SEQ ID NO : 28 |
| | SEQ ID NO : 1,SEQ ID NO : 3,SEQ ID NO : 12,SEQ ID NO : 13,SEQ ID NO : 28 |
| | SEQ ID NO : 1,SEQ ID NO : 8,SEQ ID NO : 11,SEQ ID NO : 12,SEQ ID NO : 13 |
| | SEQ ID NO : 1,SEQ ID NO : 8,SEQ ID NO : 11,SEQ ID NO : 12,SEQ ID NO : 28 |
| | SEQ ID NO : 1,SEQ ID NO : 8,SEQ ID NO : 11,SEQ ID NO : 13,SEQ ID NO : 28 |
| | SEQ ID NO : 1,SEQ ID NO : 8,SEQ ID NO : 12,SEQ ID NO : 13,SEQ ID NO : 28 |
| | SEQ ID NO : 1,SEQ ID NO : 11,SEQ ID NO : 12,SEQ ID NO : 13,SEQ ID NO : 28 |
| | SEQ ID NO : 3,SEQ ID NO : 8,SEQ ID NO : 11,SEQ ID NO : 12,SEQ ID NO : 13 |
| | SEQ ID NO : 3,SEQ ID NO : 8,SEQ ID NO : 11,SEQ ID NO : 12,SEQ ID NO : 28 |
| | SEQ ID NO : 3,SEQ ID NO : 8,SEQ ID NO : 11,SEQ ID NO : 13,SEQ ID NO : 28 |
| | SEQ ID NO : 3,SEQ ID NO : 8,SEQ ID NO : 12,SEQ ID NO : 13,SEQ ID NO : 28 |
| | SEQ ID NO : 3,SEQ ID NO : 11,SEQ ID NO : 12,SEQ ID NO : 13,SEQ ID NO : 28 |
| | SEQ ID NO : 8,SEQ ID NO : 11,SEQ ID NO : 12,SEQ ID NO : 13,SEQ ID NO : 28 |
| 6 | SEQ ID NO : 1,SEQ ID NO : 3,SEQ ID NO : 8,SEQ ID NO : 11,SEQ ID NO : 12, SEQ ID NO : 13 |
| | SEQ ID NO : 1,SEQ ID NO : 3,SEQ ID NO : 8,SEQ ID NO : 11,SEQ ID NO : 12, SEQ ID NO : 28 |
| | SEQ ID NO : 1,SEQ ID NO : 3,SEQ ID NO : 8,SEQ ID NO : 11,SEQ ID NO : 13, SEQ ID NO : 28 |
| | SEQ ID NO : 1,SEQ ID NO : 3,SEQ ID NO : 8,SEQ ID NO : 12,SEQ ID NO : 13, SEQ ID NO : 28 |
| | SEQ ID NO : 1,SEQ ID NO : 3,SEQ ID NO : 11,SEQ ID NO : 12,SEQ ID NO : 13, SEQ ID NO : 28 |
| | SEQ ID NO : 1,SEQ ID NO : 8,SEQ ID NO : 11,SEQ ID NO : 12,SEQ ID NO : 13, SEQ ID NO : 28 |
| | SEQ ID NO : 3,SEQ ID NO : 8,SEQ ID NO : 11,SEQ ID NO : 12,SEQ ID NO : 13, SEQ ID NO : 28 |
| 7 | SEQ ID NO : 1, SEQ ID NO : 3, SEQ ID NO : 8, SEQ ID NO : 11, SEQ ID NO : 12, SEQ ID NO : 13, SEQ ID NO : 28 |

Le procédé pour déterminer *in vitro* ou *ex vivo* le statut immunitaire d'un individu, tel que décrit précédemment, selon un quelconque mode de réalisation, peut également comprendre une étape de détection et/ou de quantification de l'expression, dans l'échantillon biologique test, d'au moins un, au moins 2, au moins 3, au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 21, au moins 22, au moins 23, au moins 24, au moins 25, au moins 26, au moins 27, au moins 28, au moins 29, au moins 30, au moins 31, au moins 32, au moins 33, au moins 34, au moins 35, au moins 36, au moins 37, au moins 38, au moins 39, au moins 40, au moins 41, au moins 42, au moins 43, au moins 44, au moins 45, au moins 46, au moins 47, au moins 48, au moins 49, au moins 50, au moins 51, au moins 52, au moins 53, au moins 54, au moins 55, au moins 56, au moins 57, au moins 58, au moins 59, au moins 60, au moins 61, au moins 62, au moins 63, au moins 64, au moins 65, au moins 66, au moins 67, au moins 68, au moins 69, au moins 70, au moins 71, au moins 72, au moins 73, au moins 74, au moins 75, au moins 76, au moins 77, au moins 78, au moins 79, au moins 80, au moins 81, au moins 82, au moins 83, au moins 84, au moins 85, au moins 86, au moins 87, au moins 88, au moins 89, au moins 90, au moins 91, au moins 92, au moins 93, au moins 94, au moins 95 gène(s) choisi(s) parmi les gènes suivants :
CD74, CX3CR1, IL-10, S100A8, S100A9, MERTK, CLEC7A, CD36, TIMP2, CCL13, PTGS2, IL-12B, IL-6, IL-1A, CCL20, MX1, OAS-1, CCL15, OAS-3, EIF2AK2, IFNγ, NEFH, MMP10, SERPINB2, THBD, STAT1, CCR4, HLA-DRB1/B3, TCF7, EOMES, BCL11B, ITGA7, IL-18R1, NLRC4, CYP1B1, HGF, IL-5RA, CCL5, CD3G, DPP4, CD4OLG, CD3D, CD127, ICOS, IL-1R2, IL-1RN, IL-18, IL-18RAP, OX40L, PD-1, PD-L1, Zonulin (HP), BTLA, C3AR1, CD154, GM-CSF, IFIH1, IL-15, MCP1, PCSK9, STAT4, LTR82B, CIITA, LILRB2, CD177, ADGRE3, FLT-1, CD64, TREM-1, TNF-α, IL-1β, ALOX5, IL-17A, NFκB, TBX21, HIF1a, RORgT, OAS-2, GNLY, CTLA-4, TIM 3, CD274, IL-2, IL-7R, GATA3, CXCL10, FAS, GSN, MDC1, DYRK2, TDRD9, CNB1IP1, ZAP70 et ARL14EP.

De préférence, l'invention a pour objet un procédé pour déterminer le statut immunitaire d'un individu, tel que décrit précédemment, dans lequel l'expression est détectée et/ou quantifiée au niveau transcrit ARN ou ARN messager (ARNm). La détection et/ou la quantification au niveau transcrit ARN ou ARNm peut être réalisée par tout moyen connu de l'homme du métier. En particulier, on peut citer à titre d'exemples :
- des méthodes d'hybridation, de préférence avec une puce d'hybridation, par hybridation *in situ* ou par Northern blot ;
- des méthodes d'amplification, de préférence par RT-PCR (« *Reverse Transcriptase Polymerase Chain Reaction* »), de préférence encore par RT-qPCR (RT-PCR quantitative). On peut citer en particulier la PCR emboîtée (ou PCR nichée, « *nested PCR* »). Les réactions de PCR peuvent également être multiplexées ;
- des méthodes de séquençage, de préférence par séquençage haut débit

De préférence, l'invention a pour objet un procédé pour déterminer le statut immunitaire d'un individu, tel que décrit précédemment, dans lequel le statut immunitaire est déterminé comme étant un statut d'immunosuppression (ou statut d'immunodépression ou d'immunodéficience ou statut immunitaire hypoactif ou paralysie immunitaire), un statut immunitaire normal (ou statut d'immunocompétence), ou un statut d'inflammation (ou statut immunitaire hyperactif). Le statut d'inflammation inclut le statut d'hyper-inflammation.

De préférence, l'invention a pour objet un procédé pour déterminer le statut immunitaire d'un individu, tel que décrit précédemment, dans lequel l'individu est un patient admis au sein d'une structure médicale, de préférence en unité de soins intensifs, aux urgences ou en réanimation. De préférence également, l'individu est un patient atteint de traumatismes, un patient atteint de brûlures, un patient ayant reçu une chirurgie ou un patient en état septique, de préférence un patient atteint de choc septique. De manière encore plus préférée, l'échantillon biologique test provient d'un prélèvement obtenu dans les 10 jours, de préférence dans les 9 jours, de préférence dans les 8 jours, de préférence dans les 7 jours, de préférence dans les 6 jours, de préférence dans les 5 jours, de préférence dans les 4 jours, de préférence dans les 3 jours, de préférence dans les 2 jours, de préférence dans les 24 heures, suivant l'admission au sein de la structure médicale.

Préférentiellement, l'invention a pour objet un procédé pour déterminer le statut immunitaire d'un individu, tel que décrit précédemment, dans lequel l'échantillon biologique de référence est un échantillon biologique issu d'un individu sain, de préférence un échantillon biologique issu du même individu dont est issu l'échantillon biologique test mais prélevé avant l'infection ou l'agression, ou un échantillon biologique d'un individu de statut immunitaire connu, de préférence avec un statut d'inflammation, un statut immunitaire normal, ou un statut d'immunosuppression.

De préférence, l'échantillon biologique test et/ou l'échantillon biologique de référence, tel qu'utilisé dans le procédé pour déterminer le statut immunitaire d'un individu, selon l'invention, tel que décrit précédemment, est un échantillon de sang, de préférence un échantillon de sang total, de plasma ou de sérum, ou un échantillon de cellules mononucléées sanguines périphériques, extraites à partir d'un échantillon de sang.

De préférence, le procédé pour déterminer le statut immunitaire selon l'invention, tel que décrit précédemment, comprend une étape d'administration d'un traitement, de préférence un traitement immunomodulateur, adapté au statut immunitaire de l'individu. De manière préférée, le traitement immunomodulateur est un traitement immunostimulant, s'il est déterminé que l'individu a un statut d'immunosuppression, ou un traitement anti-inflammatoire, s'il est déterminé que l'individu a un statut d'inflammation. Parmi les traitements immunostimulants qui peuvent être sélectionnés, on peut citer à titre d'exemple le groupe des interleukines, en particulier IL-7, IL-15 ou IL-3, des facteurs de croissance, en particulier le GM-CSF, des interférons, en particulier IFNγ, des Toll agonistes, des anticorps, en particulier des anticorps anti-PD1, anti-PDL1, anti-LAG3, anti-TIM3, anti-IL-10 ou anti-CTLA4, des transferrines et des molécules inhibitrices de l'apoptose, FLT3L, Thymosin α1, des antagonistes adrénergiques. Parmi les traitements anti-inflammatoires, on peut citer notamment le groupe des glucocorticoïdes, des agents cytostatiques, des molécules agissant sur les immunophilines et les cytokines, des molécules bloquant le récepteur à l'IL-1 et des traitements anti-TNF.

La présente invention a également pour objet l'utilisation d'au moins une, au moins 2, au moins 3, au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 21, au moins 22, au moins 23, au moins 24, au moins 25, au moins 26, au moins 27, au moins 28, au moins 29, au moins 30, au moins 31, au moins 32, au moins 33, au moins 34 séquence(s) choisie(s) parmi les séquences identifiées en SEQ ID NOs : 1 à 34, telles qu'apparaissant dans les Listes 1 à 4, ou parmi les séquences qui présentent au moins 99% d'identité avec une des séquences identifiées en SEQ ID NOs : 1 à 34, pour déterminer *in vitro* ou *ex vivo* le statut immunitaire d'un individu, de préférence un patient.

Sans faire partie de l'invention, la présente demande décrit un procédé pour identifier ou sélectionner un traitement, de préférence un traitement immunomodulateur, plus particulièrement un traitement immunostimulant ou un traitement anti-inflammatoire, adapté pour traiter un individu, de préférence un patient, comprenant les étapes suivantes :
a. On détermine le statut immunitaire dudit individu par un procédé tel que décrit précédemment
b. On identifie un traitement adapté à partir du statut immunitaire déterminé à l'étape a)

De manière préférée, le traitement immunomodulateur est un traitement immunostimulant, s'il est déterminé que l'individu a un statut d'immunosuppression, ou un traitement anti-inflammatoire, s'il est déterminé que l'individu a un statut d'inflammation.

Sans faire partie de l'invention, la présente demande décrit un procédé pour évaluer l'efficacité d'un traitement, de préférence un traitement immunomodulateur, plus particulièrement un traitement immunostimulant ou un traitement anti-inflammatoire, sur un individu, de préférence un patient, comprenant les étapes suivantes :
a. On détecte et/ou on quantifie l'expression d'au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, dans un premier échantillon biologique dudit individu, prélevé avant le traitement, et dans un deuxième échantillon biologique dudit individu, prélevé après le traitement
b. On compare l'expression obtenue pour les 2 échantillons biologiques à l'étape a)
c. On évalue l'efficacité du traitement à partir de la comparaison de l'étape b)

Sans faire partie de l'invention, la présente demande décrit une amorce d'amplification comprenant, ou consistant en, une séquence nucléotidique complémentaire d'au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences. De préférence, l'amorce d'amplification selon l'invention est choisie parmi les amorces présentées dans le Tableau 6.

**Tableau 6**

| **SEQ ID NO** | **Amorce #** | **Séquence nucléotidique** |
|---|---|---|
| 35 | 1A | TGTACAAAACTCAAATGGTCTTC |
| 36 | 1B | ATGACCAACTTAGATTTCCTTGA |
| 37 | 2A | GCCAGAGAGGCATAATGAAGCA |
| 38 | 2B | GATTCTAAGCCTCCCCCTCATTT |
| 39 | 3A | TGGCTCATAGGGATTCCAGACT |
| 40 | 3B | AGCAAGTTGTCAAGAGCCAATCT |
| 41 | 4A | CACTCTAGGAATCTTAGGCA |
| 42 | 4B | TGAAACCAATAGTCCAGTG |
| 43 | 5A | TTCTACTGTTCACTGCTATCCTCC |
| 44 | 5B | CCTGTGGCAGCTTTTTGAAGTAA |
| 45 | 6A | AGAGCAGAAGAAGATGGATACT |
| 46 | 6B | CATGAGCTGACATCATCCAAT |
| 47 | 7A | TCTGTACTGGTTGCCCCAAC |
| 48 | 7B | CGTGCCAGGCCTCTAATACTTTT |
| 49 | 8A | AGGGAAGACCCCAAGATGATG |
| 50 | 8B | CATGCAAAGTCCAACGAGAGG |
| 51 | 9A | GGGTGGCTGCATCCTATGG |
| 52 | 9B | CTGGTCAGGAAAAAATTTGCCTTC |
| 53 | 10A | ACATGACATTGTCTGAACTTTGGG |
| 54 | 10B | TAGGACCATGCAGATACTAGTGAC |
| 55 | 11A | GAACTCCACAAACCTTGA |
| 56 | 11B | GCTAGAAGCTTTGGATATCT |
| 57 | 12A | TGGCTGTTACAACTTTCATG |
| 58 | 12B | TCTCCCTATTCTGAGCACA |

Sans faire partie de l'invention, la présente demande décrit un couple d'amorces d'amplification, consistant en deux amorces d'amplification choisies parmi les amorces telles que décrites précédemment, et permettant d'amplifier, de préférence permettant d'amplifier spécifiquement, au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences. De préférence, le couple d'amorces d'amplification selon l'invention est choisi parmi les couples d'amorces présentés dans le Tableau 7.

**Tableau 7**

| **Couple d'amorces #** | **Amorces #** |
|---|---|
| 1 | Forward : Amorce #1A |
| | Reverse : Amorce #1B |
| 2 | Forward : Amorce #2A |
| | Reverse : Amorce #2B |
| 3 | Forward : Amorce #3A |
| | Reverse : Amorce #3B |
| 4 | Forward : Amorce #4A |
| | Reverse : Amorce #4B |
| 5 | Forward : Amorce #5A |
| | Reverse : Amorce #5B |
| 6 | Forward : Amorce #6A |
| | Reverse : Amorce #6B |
| 7 | Forward : Amorce #7A |
| | Reverse : Amorce #7B |
| 8 | Forward : Amorce #8A |
| | Reverse : Amorce #8B |
| 9 | Forward : Amorce #9A |
| | Reverse : Amorce #9B |
| 10 | Forward : Amorce #10A |
| | Reverse : Amorce #10B |
| 11 | Forward : Amorce #11A |
| | Reverse : Amorce #11B |
| 12 | Forward : Amorce #12A |
| | Reverse : Amorce #12B |

Sans faire partie de l'invention, la présente demande décrit une sonde d'hybridation, dont la séquence nucléotidique comprend, ou consiste en, une séquence nucléotidique complémentaire d'au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences. De préférence, la sonde d'hybridation selon l'invention est choisie parmi les sondes d'hybridation présentées dans le Tableau 8.

**Tableau 8**

| **SEQ ID NO** | **Sonde #** | **Nom de la sonde de la puce HERV-V3** | **Séquence nucléotidique** |
|---|---|---|---|
| 59 | 1A | 190665001-HERV0376uL_at1 | ATGACCAACTTAGATTTCCTTGAGT |
| 60 | 1B | 190665001-HERV0376uL_at2 | GTCAAGGGTAAAGCTGTGAAAGTTT |
| 61 | 1C | 190665001-HERV0376uL_at3 | GGAAGACCATTTGAGTTTTGTACAC |
| 62 | 1A' | 190665001-HERV0376uL_st1 | ACTCAAGGAAATCTAAGTTGGTCAT |
| 63 | 1B' | 190665001-HERV0376uL_st2 | AAACTTTCACAGCTTTACCCTTGAC |
| 64 | 1C' | 190665001-HERV0376uL_st3 | GTGTACAAAACTCAAATGGTCTTCC |
| 65 | 1D | 190665002-HERV0376uL_at1 | GAAGATATGGGCCAGAACTTGTATA |
| 66 | 1E | 190665002-HERV0376uL_at2 | CAGGACCTGAGTTAAGCCAAGAATA |
| 67 | 1F | 190665002-HERV0376uL_at3 | ACCTGAGTTAAGCCAAGAATACAGT |
| 68 | 1D' | 190665002-HERV0376uL_st1 | TATACAAGTTCTGGCCCATATCTTC |
| 69 | 1E' | 190665002-HERV0376uL_st2 | TATTCTTGGCTTAACTCAGGTCCTG |
| 70 | 1F' | 190665002-HERV0376uL_st3 | ACTGTATTCTTGGCTTAACTCAGGT |
| 71 | 2A | 220247002-HERV0797uL_at1 | GTAAGATTCTAAGCCTCCCCCTCAT |
| 72 | 2B | 220247002-HERV0797uL_at2 | GATTCTAAGCCTCCCCCTCATTTAA |
| 73 | 2C | 220247002-HERV0797uL_at3 | CTAAGCCTCCCCCTCATTTAAAGGA |
| 74 | 2A' | 220247002-HERV0797uL_st1 | ATGAGGGGGAGGCTTAGAATCTTAC |
| 75 | 2B' | 220247002-HERV0797uL_st2 | TTAAATGAGGGGGAGGCTTAGAATC |
| 76 | 2C' | 220247002-HERV0797uL_st3 | TCCTTTAAATGAGGGGGAGGCTTAG |
| 77 | 3A | 170369402HE41env_at1 | ATGGCTCATAGGGATTCCAGACTCC |
| 78 | 3B | 170369402HE41env_at2 | GGCTCATAGGGATTCCAGACTCCCA |
| 79 | 3C | 170369402HE41env_at3 | CTCATAGGGATTCCAGACTCCCATT |
| 80 | 3A' | 170369402HE41env_st1 | GGAGTCTGGAATCCCTATGAGCCAT |
| 81 | 3B' | 170369402HE41env_st2 | TGGGAGTCTGGAATCCCTATGAGCC |
| 82 | 3C' | 170369402HE41env_st3 | AATGGGAGTCTGGAATCCCTATGAG |
| 83 | 4A | 121601801-HERV0492uL_at1 | TGAAACCAATAGTCCAGTGGTGGCC |
| 84 | 4B | 121601801-HERV0492uL_at2 | TTCCAGTGATTTAGATAAAATCCCT |
| 85 | 4C | 121601801-HERV0492uL_at3 | TTTCTGCCTAAGATTCCTAGAGTGC |
| 86 | 4A' | 121601801-HERV0492uL_st1 | GGCCACCACTGGACTATTGGTTTCA |
| 87 | 4B' | 121601801-HERV0492uL_st2 | AGGGATTTTATCTAAATCACTGGAA |
| 88 | 4C' | 121601801-HERV0492uL_st3 | GCACTCTAGGAATCTTAGGCAGAAA |
| 89 | 5A | 011052702-MALR1044uL_at1 | CTGTGGCAGCTTTTTGAAGTAAGGA |
| 90 | 5B | 011052702-MALR1044uL_at2 | ATGGTTAGTGCAGAGTAAAGTTTGG |
| 91 | 5C | 011052702-MALR1044uL_at3 | AGGATAGCAGTGAACAGTAGAATGG |
| 92 | 5A' | 011052702-MALR1044uL_st1 | TCCTTACTTCAAAAAGCTGCCACAG |
| 93 | 5B' | 011052702-MALR1044uL_st2 | CCAAACTTTACTCTGCACTAACCAT |
| 94 | 5C' | 011052702-MALR1044uL_st3 | CCATTCTACTGTTCACTGCTATCCT |
| 95 | 6A | 011052202-HERV1033uL_at1 | AGATCCAACATGAGCTGACATCATC |
| 96 | 6A' | 011052202-HERV1033uL_st1 | GATGATGTCAGCTCATGTTGGATCT |
| 97 | 7A | 130360601-HERV0808cL_at1 | GAGGTTGGGGCAACCAGTACAGATT |
| 98 | 7A' | 130360601-HERV0808cL_st1 | AATCTGTACTGGTTGCCCCAACCTC |
| 99 | 8A | 141107102-MALR1019uL_at1 | CCCCAAGATGATGGACTCTGGTGAT |
| 100 | 8B | 141107102-MALR1019uL_at2 | CACTGCCATCACTTTGGGAAAGACT |
| 101 | 8C | 141107102-MALR1019uL_at3 | AAGCAGCCTCTCGTTGGACTTTGCA |
| 102 | 8A' | 141107102-MALR1019uL_st1 | ATCACCAGAGTCCATCATCTTGGGG |
| 103 | 8B' | 141107102-MALR1019uL_st2 | AGTCTTTCCCAAAGTGATGGCAGTG |
| 104 | 8C' | 141107102-MALR1019uL_st3 | TGCAAAGTCCAACGAGAGGCTGCTT |
| 105 | 9A | 021460102-HERV0599uL_at1 | GAGGGCAGTTTGGAACAGTTGGAAC |
| 106 | 9B | 021460102-HERV0599uL_at2 | TGAGAGACGATTATCTGGAAGAAGA |
| 107 | 9C | 021460102-HERV0599uL_at3 | TCACAGCTTGAGAATGTGGTAGGAG |
| 108 | 9D | 021460102-HERV0599uL_at4 | GGAATGGGGGGCATGGAATTAAAGC |
| 109 | 9A' | 021460102-HERV0599uL_st1 | GTTCCAACTGTTCCAAACTGCCCTC |
| 110 | 9B' | 021460102-HERV0599uL_st2 | TCTTCTTCCAGATAATCGTCTCTCA |
| 111 | 9C' | 021460102-HERV0599uL_st3 | CTCCTACCACATTCTCAAGCTGTGA |
| 112 | 9D' | 021460102-HERV0599uL_st4 | GCTTTAATTCCATGCCCCCCATTCC |
| 113 | 10A | 021456001-MALR1017uL_at1 | AGTCCCTAACTGTCTGCAAACCCAC |
| 114 | 10B | 021456001-MALR1017uL_at2 | ACTGTCTGCAAACCCACAATGGACC |
| 115 | 10C | 021456001-MALR1017uL_at3 | CAATGGACCTGTTGCATGTGTAAGA |
| 116 | 10A' | 021456001-MALR1017uL_st1 | GTGGGTTTGCAGACAGTTAGGGACT |
| 117 | 10B' | 021456001-MALR1017uL_st2 | GGTCCATTGTGGGTTTGCAGACAGT |
| 118 | 10C' | 021456001-MALR1017uL_st3 | TCTTACACATGCAACAGGTCCATTG |
| 119 | 11A | 050286701-HERV0513uL_at1 | CTGCATCCTATGGTGTTTCTACATG |
| 120 | 11B | 050286701-HERV0513uL_at2 | ATAATCTTTTCCGGCATGTTGGTAT |
| 121 | 11C | 050286701-HERV0513uL_at3 | TAAAGATAGTGTTTCCTATTGTGTC |
| 122 | 11A' | 050286701-HERV0513uL_st1 | CATGTAGAAACACCATAGGATGCAG |
| 123 | 11B' | 050286701-HERV0513uL_st2 | ATACCAACATGCCGGAAAAGATTAT |
| 124 | 11C' | 050286701-HERV0513uL_st3 | GACACAATAGGAAACACTATCTTTA |
| 125 | 12A | 050287402-MALR1022uL_at1 | ACAGAGACTGCAAGAGTAATGACAT |
| 126 | 12B | 050287402-MALR1022uL_at2 | TCTGAACTTTGGGAAACAATTATGT |
| 127 | 12C | 050287402-MALR1022uL_at3 | ACTTTCCAGTTAATCGAATCAATCC |
| 128 | 12D | 050287402-MALR1022uL_at4 | TTTTAACCTAGACTAGTTCCAACTG |
| 129 | 12E | 050287402-MALR1022uL_at5 | GTCACTAGTATCTGCATGGTCCTAA |
| 130 | 12A' | 050287402-MALR1022uL_st1 | ATGTCATTACTCTTGCAGTCTCTGT |
| 131 | 12B' | 050287402-MALR1022uL_st2 | ACATAATTGTTTCCCAAAGTTCAGA |
| 132 | 12C' | 050287402-MALR1022uL_st3 | GGATTGATTCGATTAACTGGAAAGT |
| 133 | 12 D' | 050287402-MALR1022uL_st4 | CAGTTGGAACTAGTCTAGGTTAAAA |
| 134 | 12E' | 050287402-MALR1022uL_st5 | TTAGGACCATGCAGATACTAGTGAC |
| 135 | 13A | 052182701-MALR1129uL_at1 | TTATTCCAGTCACCTCGAGTCATTC |
| 136 | 13B | 052182701-MALR1129uL_at2 | TCATCCTAGCCGTCGTAGAGCAGAG |
| 137 | 13C | 052182701-MALR1129uL_at3 | TGCCCTTCTGACTCCTTGACAGTGG |
| 138 | 13A' | 052182701-MALR1129uL_st1 | GAATGACTCGAGGTGACTGGAATAA |
| 139 | 13B' | 052182701-MALR1129uL_st2 | CTCTGCTCTACGACGGCTAGGATGA |
| 140 | 13C' | 052182701-MALR1129uL_st3 | CCACTGTCAAGGAGTCAGAAGGGCA |
| 141 | 14A | 190478501-MALR1003cL_at1 | TAAGTGGGACCAAGACACAAACCAA |
| 142 | 14B | 190478501-MALR1003cL_at3 | ACCAAGACACAAACCAACATGCCTG |
| 143 | 14A' | 190478501-MALR1003cL_st1 | TTGGTTTGTGTCTTGGTCCCACTTA |
| 144 | 14B' | 190478501-MALR1003cL_st3 | CAGGCATGTTGGTTTGTGTCTTGGT |
| 145 | 15A | 011790601ERV9sLU5p_at1 | CTGAGGTCCATGGCTTCTTTCCTTG |
| 146 | 15A' | 011790601ERV9sLU5p_st1 | CAAGGAAAGAAGCCATGGACCTCAG |
| 147 | 16A | 052681601-MALR1018uL_at1 | CCTTTGTTTTCCTACTGACAGGTCC |
| 148 | 16B | 052681601-MALR1018uL_at2 | TTCAAAATATTTAACTCTCCAGGCT |
| 149 | 16C | 052681601-MALR1018uL_at3 | GAGGTCACATGACTCTGTTGTGGAC |
| 150 | 16A' | 052681601-MALR1018uL_st1 | GGACCTGTCAGTAGGAAAACAAAGG |
| 151 | 16B' | 052681601-MALR1018uL_st2 | AGCCTGGAGAGTTAAATATTTTGAA |
| 152 | 16C' | 052681601-MALR1018uL_st3 | GTCCACAACAGAGTCATGTGACCTC |
| 153 | 17A | 160627301-MALR1014uL_at1 | CAGCTGAGATCCGTTGACGCCAGCC |
| 154 | 17B | 160627301-MALR1014uL_at2 | TCCGACATGTGGGTGAACTCAGCCA |
| 155 | 17C | 160627301-MALR1014uL_at3 | TTCTCAGCCATGTGTTTTGTGAACT |
| 156 | 17A' | 160627301-MALR1014uL_st1 | GGCTGGCGTCAACGGATCTCAGCTG |
| 157 | 17B' | 160627301-MALR1014uL_st2 | TGGCTGAGTTCACCCACATGTCGGA |
| 158 | 17C' | 160627301-MALR1014uL_st3 | AGTTCACAAAACACATGGCTGAGAA |
| 159 | 18A | 111686702-HERV0861uL_at1 | TTGAGGCAGGACAGAACCAGGCTCC |
| 160 | 18B | 111686702-HERV0861uL_at2 | GGACAGAACCAGGCTCCTGTTAGTC |
| 161 | 18C | 111686702-HERV0861uL_at3 | AGTTTACTGAGCAGTGACTTTGTGT |
| 162 | 18A' | 111686702-HERV0861uL_st1 | GGAGCCTGGTTCTGTCCTGCCTCAA |
| 163 | 18B' | 111686702-HERV0861uL_st2 | GACTAACAGGAGCCTGGTTCTGTCC |
| 164 | 18C' | 111686702-HERV0861uL_st3 | ACACAAAGTCACTGCTCAGTAAACT |
| 165 | 19A | 040318302-MALR1134uL_at1 | ATAGGGATGATCCTGCACGAATGGC |
| 166 | 19B | 040318302-MALR1134uL_at2 | GGATGATCCTGCACGAATGGCATGG |
| 167 | 19A' | 040318302-MALR1134uL_st1 | GCCATTCGTGCAGGATCATCCCTAT |
| 168 | 19B' | 040318302-MALR1134uL_st2 | CCATGCCATTCGTGCAGGATCATCC |
| 169 | 20A | 041529101-MALR1026uL_at1 | AGTGGACACTTTTTAGGATGTCTGC |
| 170 | 20B | 041529101-MALR1026uL_at2 | GCCCTGACATAAGAGTTTGCCAGTT |
| 171 | 20C | 041529101-MALR1026uL_at3 | CCTGTACCCACCTTTCACCAGAGCT |
| 172 | 20A' | 041529101-MALR1026uL_st1 | GCAGACATCCTAAAAAGTGTCCACT |
| 173 | 20B' | 041529101-MALR1026uL_st2 | AACTGGCAAACTCTTATGTCAGGGC |
| 174 | 20C' | 041529101-MALR1026uL_st3 | AGCTCTGGTGAAAGGTGGGTACAGG |
| 175 | 21A | 141106902-MALR1133uL_at1 | AATTGTTGGAATTTGAAAGTGGGGT |
| 176 | 21A' | 141106902-MALR1133uL_st1 | ACCCCACTTTCAAATTCCAACAATT |
| 177 | 22A | 060281701-MALR1043uL_at1 | GTCAGCACCGTGCTTCTCTAACTTT |
| 178 | 22B | 060281701-MALR1043uL_at2 | GCACCGTGCTTCTCTAACTTTCCAC |
| 179 | 22C | 060281701-MALR1043uL_at3 | CGTGCTTCTCTAACTTTCCACCTGC |
| 180 | 22A' | 060281701-MALR1043uL_st1 | AAAGTTAGAGAAGCACGGTGCTGAC |
| 181 | 22B' | 060281701-MALR1043uL_st2 | GTGGAAAGTTAGAGAAGCACGGTGC |
| 182 | 22C' | 060281701-MALR1043uL_st3 | GCAGGTGGAAAGTTAGAGAAGCACG |
| 183 | 23A | 043166601-MALR1018uL_at1 | CAGCCTCGCACCTAAGAACGCCGTG |
| 184 | 23B | 043166601-MALR1018uL_at2 | CAGTGAGAAATCTGCTGGGGATGCC |
| 185 | 23C | 043166601-MALR1018uL_at3 | GAAAGGGACATACCTGGCAGGTGCC |
| 186 | 23A' | 043166601-MALR1018uL_st1 | CACGGCGTTCTTAGGTGCGAGGCTG |
| 187 | 23B' | 043166601-MALR1018uL_st2 | GGCATCCCCAGCAGATTTCTCACTG |
| 188 | 23C' | 043166601-MALR1018uL_st3 | GGCACCTGCCAGGTATGTCCCTTTC |
| 189 | 24A | 100090601-HERV0429uL_at1 | GGTAGAGACCGAGGCGGATATACAG |
| 190 | 24B | 100090601-HERV0429uL_at3 | GAGACCGAGGCGGATATACAGGCCT |
| 191 | 24A' | 100090601-HERV0429uL_st1 | CTGTATATCCGCCTCGGTCTCTACC |
| 192 | 24B' | 100090601-HERV0429uL_st3 | AGGCCTGTATATCCGCCTCGGTCTC |
| 193 | 25A | 061529601-HERV0492uL_at1 | TATACTGGGGCCCAATTCTACAGAC |
| 194 | 25B | 061529601-HERV0492uL_at2 | CAGACATTACTTCTTTGCCAGTTGG |
| 195 | 25C | 061529601-HERV0492uL_at3 | GACACATTGCAAGTCTGGAAGAGGA |
| 196 | 25A' | 061529601-HERV0492uL_st1 | GTCTGTAGAATTGGGCCCCAGTATA |
| 197 | 25B' | 061529601-HERV0492uL_st2 | CCAACTGGCAAAGAAGTAATGTCTG |
| 198 | 25C' | 061529601-HERV0492uL_st3 | TCCTCTTCCAGACTTGCAATGTGTC |
| 199 | 26A | 100871501-MALR1020cL_at1 | CATGATCCTGGGTGAAGCCATGTGT |
| 200 | 26B | 100871501-MALR1020cL_at2 | TGTGTCTGAGGATGAAAGGGGATGC |
| 201 | 26C | 100871501-MALR1020cL_at3 | CAGATTGATGTGACATGTGGCACCT |
| 202 | 26A' | 100871501-MALR1020cL_st1 | ACACATGGCTTCACCCAGGATCATG |
| 203 | 26B' | 100871501-MALR1020cL_st2 | GCATCCCCTTTCATCCTCAGACACA |
| 204 | 26C' | 100871501-MALR1020cL_st3 | AGGTGCCACATGTCACATCAATCTG |
| 205 | 27A | 170842002-MALR1003uL_at1 | AGAGGGAGCACGGTCCCAGTACACC |
| 206 | 27B | 170842002-MALR1003uL_at2 | CACGGTCCCAGTACACCTTGAGTGT |
| 207 | 27C | 170842002-MALR1003uL_at3 | TGTTACGGCTGTCCCAGGAAAGGAA |
| 208 | 27A' | 170842002-MALR1003uL_st1 | GGTGTACTGGGACCGTGCTCCCTCT |
| 209 | 27B' | 170842002-MALR1003uL_st2 | ACACTCAAGGTGTACTGGGACCGTG |
| 210 | 27C' | 170842002-MALR1003uL_st3 | TTCCTTTCCTGGGACAGCCGTAACA |
| 211 | 28A | 081921103-HERV0958ul_at1 | ACTAAGAGCAACAGCCTGAGGCTAA |
| 212 | 28B | 081921103-HERV0958ul_at2 | GGCTCACCGGAAACAGGCTGAATGT |
| 213 | 28C | 081921103-HERV0958ul_at3 | GAGACACCAGATGACCGCTTGGTCT |
| 214 | 28D | 081921103-HERV0958ul_at4 | CAGCTTCCCTAGAATTATACACCAG |
| 215 | 28E | 081921103-HERV0958ul_at5 | TACTGAACAGGTTACTTCAACTTGC |
| 216 | 28F | 081921103-HERV0958ul_at6 | TTGTAAAAATATAAACGTGAGGCAA |
| 217 | 28A' | 081921103-HERV0958ul_st1 | TTAGCCTCAGGCTGTTGCTCTTAGT |
| 218 | 28B' | 081921103-HERV0958ul_st2 | ACATTCAGCCTGTTTCCGGTGAGCC |
| 219 | 28C' | 081921103-HERV0958ul_st3 | AGACCAAGCGGTCATCTGGTGTCTC |
| 220 | 28D' | 081921103-HERV0958ul_st4 | CTGGTGTATAATTCTAGGGAAGCTG |
| 221 | 28E' | 081921103-HERV0958ul_st5 | GCAAGTTGAAGTAACCTGTTCAGTA |
| 222 | 28F' | 081921103-HERV0958ul_st6 | TTGCCTCACGTTTATATTTTTACAA |
| 223 | 28G | 081921101-HERV0958ul_at1 | GATGACAGTTAAGACCCTAGGTTGC |
| 224 | 28H | 081921101-HERV0958ul_at2 | CAATCTCAAGTCTGATGACTTGTTA |
| 225 | 28I | 081921101-HERV0958ul_at3 | AGACCCATCATTGCTAGCAGACTAT |
| 226 | 28J | 081921101-HERV0958ul_at4 | AAGGATGGGAAATGCTCAGGTCACG |
| 227 | 28K | 081921101-HERV0958ul_at5 | AGGGCTCATCCACTAACCCCCTGAA |
| 228 | 28L | 081921101-HERV0958ul_at6 | GAAATGGATACCCTTGGGTTCAACT |
| 229 | 28G' | 081921101-HERV0958ul_st1 | GCAACCTAGGGTCTTAACTGTCATC |
| 230 | 28H' | 081921101-HERV0958ul_st2 | TAACAAGTCATCAGACTTGAGATTG |
| 231 | 28I' | 081921101-HERV0958ul_st3 | ATAGTCTGCTAGCAATGATGGGTCT |
| 232 | 28J' | 081921101-HERV0958ul_st4 | CGTGACCTGAGCATTTCCCATCCTT |
| 233 | 28K' | 081921101-HERV0958ul_st5 | TTCAGGGGGTTAGTGGATGAGCCCT |
| 234 | 28L' | 081921101-HERV0958ul_st6 | AGTTGAACCCAAGGGTATCCATTTC |
| 235 | 28M | 081921102-HERV0958ul_at1 | GTGCCATAACGACAATTAAATTTTT |
| 236 | 28N | 081921102-HERV0958ul_at2 | AGTCTTTTGTTATCTATGGAGGACT |
| 237 | 28O | 081921102-HERV0958ul_at3 | GTTGTGTTAAAGTTTCTAATTACG |
| 238 | 28P | 081921102-HERV0958ul_at4 | GTAACTTTGGGACCAAAACAATGAA |
| 239 | 28Q | 081921102-HERV0958ul_at5 | TCATAAGCCTACTAATCCGGGATCA |
| 240 | 28R | 081921102-HERV0958ul_at6 | GGGACAAGAACTAATTCCACAGGAG |
| 241 | 28M' | 081921102-HERV0958ul_st1 | AAAAATTTAATTGTCGTTATGGCAC |
| 242 | 28N' | 081921102-HERV0958ul_st2 | AGTCCTCCATAGATAACAAAAGACT |
| 243 | 28O' | 081921102-HERV0958ul_st3 | ACGTAATTAGAAACTTTAACACAAC |
| 244 | 28P' | 081921102-HERV0958ul_st4 | TTCATTGTTTTGGTCCCAAAGTTAC |
| 245 | 28Q' | 081921102-HERV0958ul_st5 | TGATCCCGGATTAGTAGGCTTATGA |
| 246 | 28R' | 081921102-HERV0958ul_st6 | CTCCTGTGGAATTAGTTCTTGTCCC |
| 247 | 29A | 032622601MR41sLU5p_at1 | GCCCTTTCTTGAGGTCTGGGTCTGC |
| 248 | 29A' | 032622601MR41sLU5p_st1 | GCAGACCCAGACCTCAAGAAAGGGC |
| 249 | 30A | 220246901-HERV0889uL_at1 | AGGCTGTAACCCCCCTTAAACTGCC |
| 250 | 30B | 220246901-HERV0889uL_at2 | CAACTATGGGGAACTTAACTGGAGT |
| 251 | 30C | 220246901-HERV0889uL_at3 | GGAGTCGTTTCAGATGGGTGCTTAC |
| 252 | 30A' | 220246901-HERV0889uL_st1 | GGCAGTTTAAGGGGGGTTACAGCC |
| 253 | 30B' | 220246901-HERV0889uL_st2 | ACTCCAGTTAAGTTCCCCATAGTTG |
| 254 | 30C' | 220246901-HERV0889uL_st3 | GTAAGCACCCATCTGAAACGACTCC |
| 255 | 31A | 061827101-HERV0856uL_at1 | GAAGAGTTTCAGCCCTTCAGACAAC |
| 256 | 31B | 061827101-HERV0856uL_at2 | GATCCAGGTTTGTCACGCAAGCTGA |
| 257 | 31C | 061827101-HERV0856uL_at3 | CCGAGTGGGCACATCAAGCACAGTG |
| 258 | 31A' | 061827101-HERV0856uL_st1 | GTTGTCTGAAGGGCTGAAACTCTTC |
| 259 | 31B' | 061827101-HERV0856uL_st2 | TCAGCTTGCGTGACAAACCTGGATC |
| 260 | 31C' | 061827101-HERV0856uL_st3 | CACTGTGCTTGATGTGCCCACTCGG |
| 261 | 32A | 170828901-HERV0770cL_at1 | CACAGGTCTTGCCGAGACCCCCACG |
| 262 | 32B | 170828901-HERV0770cL_at2 | CCACGGGCTCACTGTTCAGCTCATC |
| 263 | 32C | 170828901-HERV0770cL_at3 | GCTCTGTCACAGTTTCCCACGACTT |
| 264 | 32A' | 170828901-HERV0770cL_st1 | CGTGGGGGTCTCGGCAAGACCTGTG |
| 265 | 32B' | 170828901-HERV0770cL_st2 | GATGAGCTGAACAGTGAGCCCGTGG |
| 266 | 32C' | 170828901-HERV0770cL_st3 | AAGTCGTGGGAAACTGTGACAGAGC |
| 267 | 33A | 190148802-MALR1127uL_at1 | GGCCAAATTGTGCCACCCCTCCCAA |
| 268 | 33B | 190148802-MALR1127uL_at2 | AATTCCCAGGACCTCCTAATATGGC |
| 269 | 33C | 190148802-MALR1127uL_at3 | GGTCGTTGTAGGCCCAGCACAGTGG |
| 270 | 33A' | 190148802-MALR1127uL_st1 | TTGGGAGGGGTGGCACAATTTGGCC |
| 271 | 33B' | 190148802-MALR1127uL_st2 | GCCATATTAGGAGGTCCTGGGAATT |
| 272 | 33C' | 190148802-MALR1127uL_st3 | CCACTGTGCTGGGCCTACAACGACC |
| 273 | 34A | 120093401-HERV1034uL_at1 | CCTAGCCATGAGCCAATTCCTTGCA |
| 274 | 34A' | 120093401-HERV1034uL_st1 | TGCAAGGAATTGGCTCATGGCTAGG |

Sans faire partie de l'invention, la présente demande décrit l'utilisation d'au moins un, de préférence au moins 2, de préférence au moins 3, de préférence au moins 4, de préférence au moins 5, de préférence au moins 6, de préférence au moins 7, de préférence au moins 8, de préférence au moins 9, de préférence au moins 10, de préférence au moins 11, de préférence au moins 12, de préférence au moins 13, de préférence au moins 14, de préférence au moins 15, de préférence au moins 16, de préférence au moins 17, de préférence au moins 18, de préférence au moins 19, de préférence au moins 20, de préférence au moins 21, de préférence au moins 22, de préférence au moins 23, de préférence au moins 24, de préférence au moins 25, de préférence au moins 26, de préférence au moins 27, de préférence au moins 28, de préférence au moins 29, de préférence au moins 30, de préférence au moins 31, de préférence au moins 32, de préférence au moins 33, de préférence au moins 34 couples d'amorces selon l'invention, tels que décrits précédemment,
et/ou d'au moins une, de préférence au moins 2, de préférence au moins 3, de préférence au moins 4, de préférence au moins 5, de préférence au moins 6, de préférence au moins 7, de préférence au moins 8, de préférence au moins 9, de préférence au moins 10, de préférence au moins 11, de préférence au moins 12, de préférence au moins 13, de préférence au moins 14, de préférence au moins 15, de préférence au moins 16, de préférence au moins 17, de préférence au moins 18, de préférence au moins 19, de préférence au moins 20, de préférence au moins 21, de préférence au moins 22, de préférence au moins 23, de préférence au moins 24, de préférence au moins 25, de préférence au moins 26, de préférence au moins 27, de préférence au moins 28, de préférence au moins 29, de préférence au moins 30, de préférence au moins 31, de préférence au moins 32, de préférence au moins 33, de préférence au moins 34 sondes d'hybridation selon l'invention, tels que décrits précédemment,
pour déterminer *in vitro* ou *ex vivo* le statut immunitaire d'un individu, de préférence un patient.

Sans faire partie de l'invention, la présente demande décrit un procédé pour déterminer *in vitro* ou *ex vivo* le statut immunitaire d'un individu, tel que décrit précédemment, dans lequel on utilise au moins un, de préférence au moins 2, de préférence au moins 3, de préférence au moins 4, de préférence au moins 5, de préférence au moins 6, de préférence au moins 7, de préférence au moins 8, de préférence au moins 9, de préférence au moins 10, de préférence au moins 11, de préférence au moins 12, de préférence au moins 13, de préférence au moins 14, de préférence au moins 15, de préférence au moins 16, de préférence au moins 17, de préférence au moins 18, de préférence au moins 19, de préférence au moins 20, de préférence au moins 21, de préférence au moins 22, de préférence au moins 23, de préférence au moins 24, de préférence au moins 25, de préférence au moins 26, de préférence au moins 27, de préférence au moins 28, de préférence au moins 29, de préférence au moins 30, de préférence au moins 31, de préférence au moins 32, de préférence au moins 33, de préférence au moins 34 couples d'amorces tels que décrits précédemment ;
et/ou au moins une, de préférence au moins 2, de préférence au moins 3, de préférence au moins 4, de préférence au moins 5, de préférence au moins 6, de préférence au moins 7, de préférence au moins 8, de préférence au moins 9, de préférence au moins 10, de préférence au moins 11, de préférence au moins 12, de préférence au moins 13, de préférence au moins 14, de préférence au moins 15, de préférence au moins 16, de préférence au moins 17, de préférence au moins 18, de préférence au moins 19, de préférence au moins 20, de préférence au moins 21, de préférence au moins 22, de préférence au moins 23, de préférence au moins 24, de préférence au moins 25, de préférence au moins 26, de préférence au moins 27, de préférence au moins 28, de préférence au moins 29, de préférence au moins 30, de préférence au moins 31, de préférence au moins 32, de préférence au moins 33, de préférence au moins 34 sondes d'hybridation telles que décrites précédemment.

Un autre objet de l'invention est l'utilisation d'un kit pour déterminer *in vitro* ou *ex vivo* le statut immunitaire d'un individu, de préférence un patient, comprenant des moyens d'amplification et/ou de détection d'au moins une, de préférence au moins 2, de préférence au moins 3, de préférence au moins 4, de préférence au moins 5, de préférence au moins 6, de préférence au moins 7, de préférence au moins 8, de préférence au moins 9, de préférence au moins 10, de préférence au moins 11, de préférence au moins 12, de préférence au moins 13, de préférence au moins 14, de préférence au moins 15, de préférence au moins 16, de préférence au moins 17, de préférence au moins 18, de préférence au moins 19, de préférence au moins 20, de préférence au moins 21, de préférence au moins 22, de préférence au moins 23, de préférence au moins 24, de préférence au moins 25, de préférence au moins 26, de préférence au moins 27, de préférence au moins 28, de préférence au moins 29, de préférence au moins 30, de préférence au moins 31, de préférence au moins 32, de préférence au moins 33, de préférence au moins 34 séquences choisies parmi les séquences identifiées en SEQ ID NOs : 1 à 34 ou parmi les séquences qui présentent au moins 99% d'identité avec une des séquences identifiées en SEQ ID NOs : 1 à 34.

De manière préférée, dans le kit:
les moyens d'amplification comprennent, de préférence consistent en au moins un, de préférence au moins 2, de préférence au moins 3, de préférence au moins 4, de préférence au moins 5, de préférence au moins 6, de préférence au moins 7, de préférence au moins 8, de préférence au moins 9, de préférence au moins 10, de préférence au moins 11, de préférence au moins 12, de préférence au moins 13, de préférence au moins 14, de préférence au moins 15, de préférence au moins 16, de préférence au moins 17, de préférence au moins 18, de préférence au moins 19, de préférence au moins 20, de préférence au moins 21, de préférence au moins 22, de préférence au moins 23, de préférence au moins 24, de préférence au moins 25, de préférence au moins 26, de préférence au moins 27, de préférence au moins 28, de préférence au moins 29, de préférence au moins 30, de préférence au moins 31, de préférence au moins 32, de préférence au moins 33, de préférence au moins 34 couples d'amorces tels que décrits précédemment ;
et/ou les moyens de détection comprennent, de préférence consistent en, au moins une, de préférence au moins 2, de préférence au moins 3, de préférence au moins 4, de préférence au moins 5, de préférence au moins 6, de préférence au moins 7, de préférence au moins 8, de préférence au moins 9, de préférence au moins 10, de préférence au moins 11, de préférence au moins 12, de préférence au moins 13, de préférence au moins 14, de préférence au moins 15, de préférence au moins 16, de préférence au moins 17, de préférence au moins 18, de préférence au moins 19, de préférence au moins 20, de préférence au moins 21, de préférence au moins 22, de préférence au moins 23, de préférence au moins 24, de préférence au moins 25, de préférence au moins 26, de préférence au moins 27, de préférence au moins 28, de préférence au moins 29, de préférence au moins 30, de préférence au moins 31, de préférence au moins 32, de préférence au moins 33, de préférence au moins 34 sondes d'hybridation telles que décrites précédemment.

Préférentiellement encore, le kit utilisé pour la présente invention comprend en outre des moyens d'amplification et/ou de détection d'autres biomarqueurs, en particulier des biomarqueurs endogènes (ou loci), tels que d'autres HERV/MaLR et/ou des gènes, de préférence des gènes impliqués dans l'inflammation et/ou l'immunité, et/ou des gènes de ménage, et/ou des biomarqueurs exogènes, tels que des virus. Parmi les gènes impliqués dans l'immunité, on peut notamment citer les gènes suivants, particulièrement d'intérêt :
CD74, CX3CR1, IL-10, S100A8, S100A9, MERTK, CLEC7A, CD36, TIMP2, CCL13, PTGS2, IL-12B, IL-6, IL-1A, CCL20, MX1, OAS-1, CCL15, OAS-3, EIF2AK2, IFNγ, NEFH, MMP10, SERPINB2, THBD, STAT1, CCR4, HLA-DRB1/B3, TCF7, EOMES, BCL11B, ITGA7, IL-18R1, NLRC4, CYP1B1, HGF, IL-5RA, CCL5, CD3G, DPP4, CD4OLG, CD3D, CD127, ICOS IL-1R2, IL-1RN, IL-18, IL-18RAP, OX40L, PD-1, PD-L1, Zonulin (HP), BTLA, C3AR1, CD154, GM-CSF, IFIH1, IL-15, MCP1, PCSK9, STAT4, LTR82B CIITA, LILRB2, CD177, ADGRE3, FLT-1, CD64, TREM-1, TNF-α, IL-1β, ALOX5, IL-17A, NFκB, TBX21, HIF1a, RORgT, OAS-2, GNLY, CTLA-4, TIM 3, CD274, IL-2, IL-7R, GATA3, CXCL10, FAS, GSN, MDC1, DYRK2, TDRD9, CNB1IP1, ZAP70 et ARL14EP.

De manière encore plus préférée, le kit utilisé pour la présente invention comprend des moyens d'amplification et/ou de détection d'au plus 100, de préférence au plus 90, de préférence au plus 80, de préférence au plus 70, de préférence au plus 60, de préférence au plus 50, de préférence au plus 40, de préférence au plus 30, de préférence au plus 20, de préférence au plus 10 biomarqueurs, au total.

### Figures

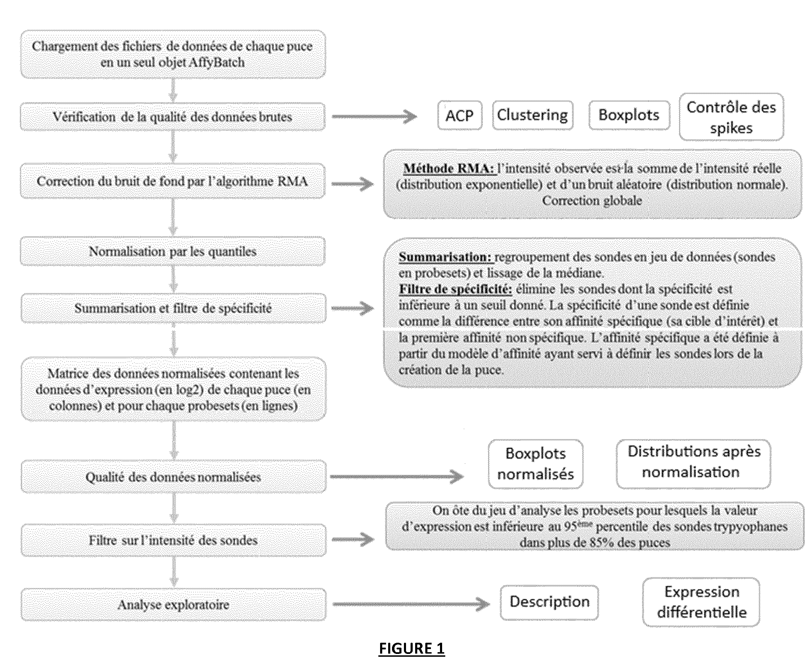

Figure 1 : Pipeline d'analyse de la puce HERV-V3. Ce schéma représente les différentes étapes nécessaires à l'analyse des données de la puce HERV-V3.

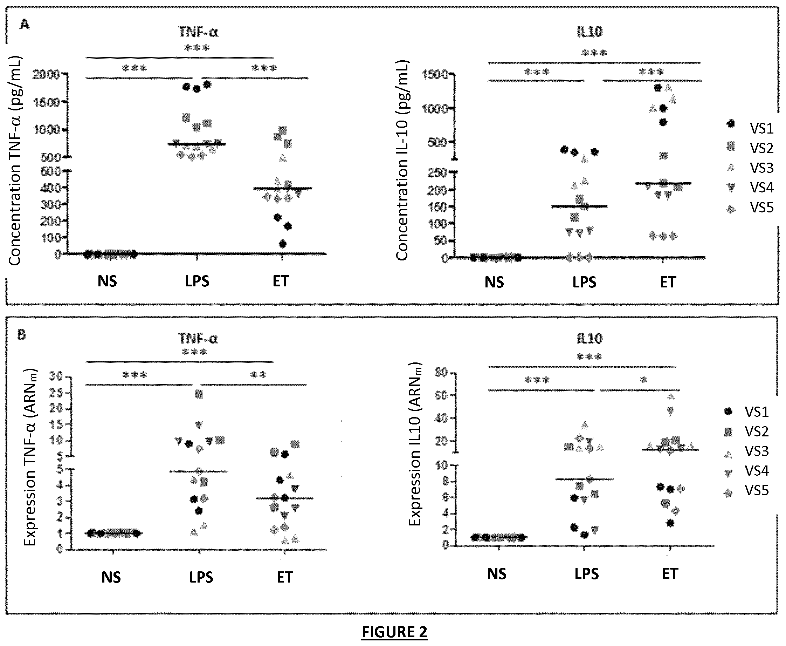

Figure 2 : Les figures 2A et 2B représentent respectivement le niveau d'expression protéique et moléculaire (ARNₘ) de TNF-α et d'IL-10 produites par des PBMC, issues de 5 volontaires sains, stimulées par du LPS. Le dosage des protéines a été effectué par un test ELISA sur des surnageants de culture obtenus après réalisation du modèle de tolérance à l'endotoxine. Dans la figure 2A, l'axe des ordonnées représente les concentrations protéiques (pg/mL) de TNF-α et d'IL-10. Dans la figure 2B, l'axe des ordonnées représente les niveaux d'expression de TNF-α et d'IL-10 (en ratio d'expression ou Fold Change (FC)). Les trois conditions sont représentées, NS pour les contrôles négatifs (sans stimulation), LPS pour les cellules stimulées une seule fois avec 100 ng/mL de LPS et ET pour les cellules soumises à deux stimulations par du LPS (2 ng/mL puis 100 ng/mL). Des tests appariés de Wilcoxon ont été réalisés pour l'analyse statistique des résultats : ***, signifie que la p-value<0,01 entre 2 conditions (NS vs. LPS ou NS vs. ET ou LPS vs. ET). **, signifie que la p-value<0,05 entre 2 conditions. *, signifie que la p-value<0,1 entre 2 conditions.

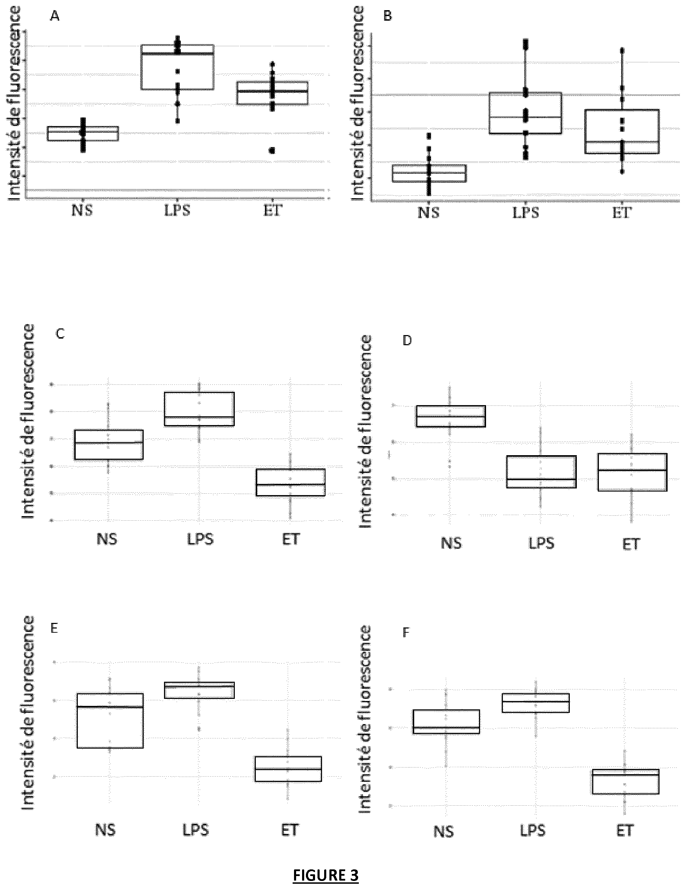

Figure 3 : La figure 3 montre l'expression des gènes de TNF-α (A) et IL-10 (B), et des séquences SEQ ID NO 1 (C), SEQ ID NO 4 (D), SEQ ID NO 5 (E) et SEQ ID NO 6 (F), par des PBMC, issues de 5 volontaires sains, stimulées par du LPS et quantifiées par biopuces après la réalisation du modèle de tolérance à l'endotoxine. L'axe des ordonnées représente l'intensité de fluorescence de chacune des sondes d'hybridation : (A) 207113_s_at pour le TNF alpha, (B) 207433_at pour l'IL-10, (C) 190665001-HERV0376uL_at pour SEQ ID NO 1, (D) 121601801-HERV0492uL_at pour SEQ ID NO 4, (E) 011052702-MALR1044uL_at pour SEQ ID NO 5 et (F) 011052202-HERV1033uL_at pour SEQ ID NO 6. Les trois conditions sont représentées, NS pour les contrôles négatifs (sans stimulation), LPS pour cellules stimulées une seule fois avec 100 ng/mL de LPS et ET pour les cellules soumises à deux stimulations par du LPS (2 ng/mL puis 100 ng/mL).

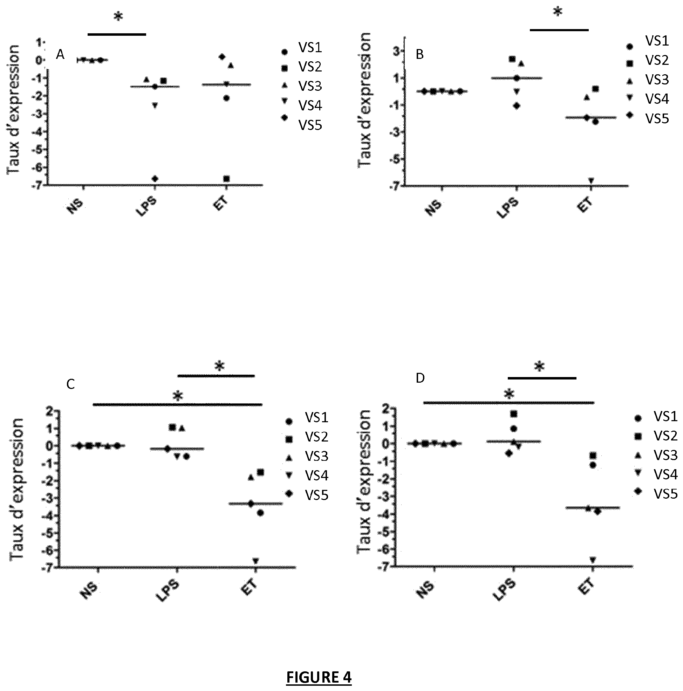

Figure 4 : La figure 4 montre l'expression des séquences SEQ ID NO 1 (C), SEQ ID NO 4 (D), SEQ ID NO 5 (E) , SEQ ID NO 6 (F), par des PBMC, issues de 5 volontaires sains, stimulées par du LPS et quantifiées par RT-qPCR après réalisation du modèle de tolérance à l'endotoxine. L'axe des ordonnées représente les taux d'expression des séquences citées ci-dessus. Les trois conditions sont représentées, NS pour les contrôles négatifs (sans stimulation), LPS pour cellules stimulées une seule fois avec 100 ng/mL de LPS et ET pour les cellules soumises à deux stimulations par du LPS (2 ng/mL puis 100 ng/mL). Des tests appariés de Wilcoxon ont été réalisés pour l'analyse statistique des résultats : ***, signifie que la p-value<0,01 entre 2 conditions (NS vs. LPS ou NS vs. ET ou LPS vs. ET). **, signifie que la p-value<0,05 entre 2 conditions. *, signifie que la p-value<0,1 entre 2 conditions.

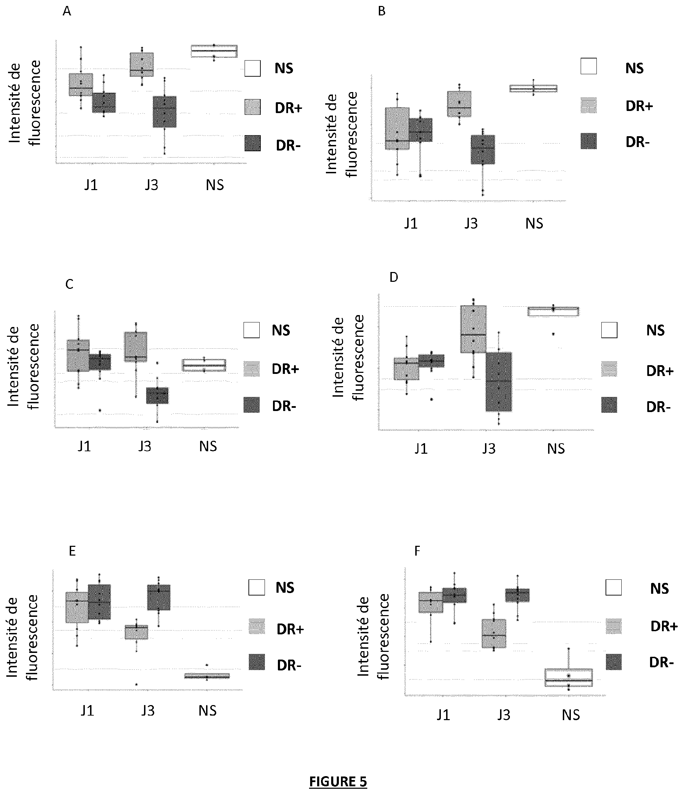

Figure 5 : La figure 5 montre l'expression des séquences SEQ ID NO 2 (A), SEQ ID NO 3 (B), SEQ ID NO 7 (C), SEQ ID NO 8 (D), SEQ ID NO 11 (E) et SEQ ID NO 12 (F), à partir de sang total de 20 patients en choc septique stratifiés en fonction du niveau d'expression de mHLA-DR et quantifiées par biopuces. L'axe des ordonnées représente l'intensité de fluorescence de chacune des sondes d'hybridation : (A) 220247002-HERV0797uL pour SEQ ID NO 2, (B) 170369402HE41env pour SEQ ID NO 3, (C) 130360601-HERV0808cL pour SEQ ID NO 7, (D) 141107102-MALR1019uL pour SEQ ID NO 8, (E) 050286701-HERV0513uL pour SEQ ID NO 11 et (F) 050287402-MALR1022uL pour SEQ ID NO 12. Les trois conditions sont représentées, VS pour les volontaires sains, et à J1 et J3, DR+ pour les patients, ayant une expression élevée de HLA-DR, considérés comme immunocompétents et DR- les patients, ayant une expression faible de HLA-DR, considérés comme immunodéprimés.

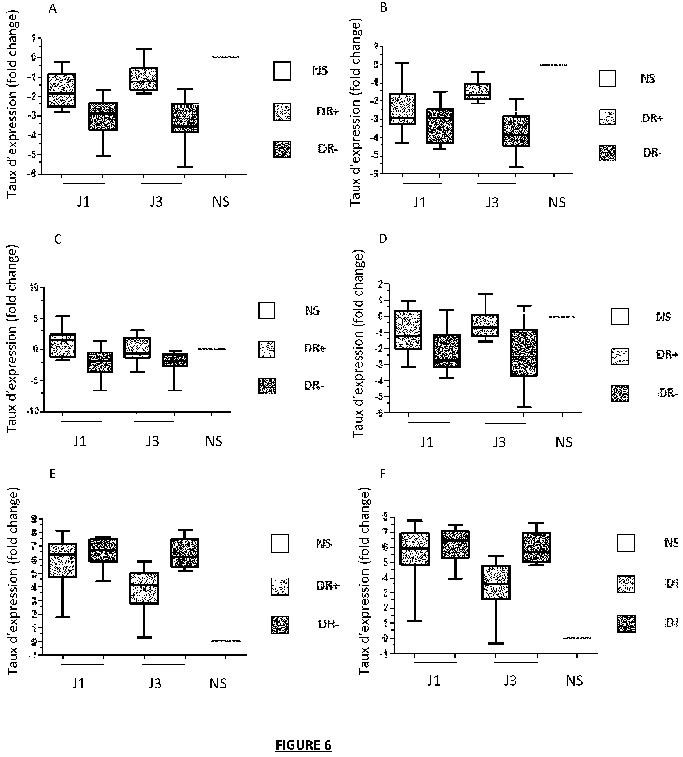

Figure 6 : La figure 6 montre l'expression des séquences SEQ ID NO 2 (A), SEQ ID NO 3 (B), SEQ ID NO 7 (C), SEQ ID NO 8 (D), SEQ ID NO 11 (E) et SEQ ID NO 12 (F), à partir de sang total de 20 patients en choc septique stratifiés en fonction du niveau d'expression de mHLA-DR et quantifiées par RT-qPCR. L'axe des ordonnées représente les taux d'expression des séquences citées ci-dessus. Les trois conditions sont représentées, VS pour les volontaires sains, et à J1 et J3, DR+ pour les patients, ayant une expression élevée de HLA-DR, considérés comme immunocompétents et DR- les patients, ayant une expression faible de HLA-DR, considérés comme immunodéprimés.

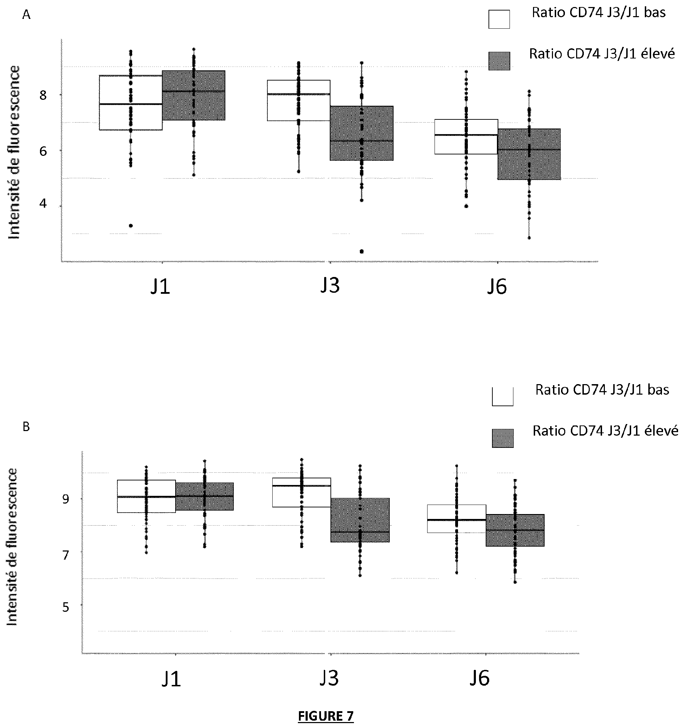

Figure 7 : La figure 7 montre l'expression des séquences SEQ ID NO 9 (A) et SEQ ID NO 10 (B), à partir de sang total de 102 patients en choc septique stratifiés en fonction du ratio entre le niveau d'expression de CD74 à J3 et le niveau d'expression de CD74 à J1 et quantifiées par biopuces. L'axe des ordonnées représente l'intensité de fluorescence de chacune des sondes d'hybridation : (A) 021460102-HERV0599uL_st pour SEQ ID NO 9 et (B) 021456001-MALR1017uL_at pour SEQ ID NO 10. Les conditions suivantes sont représentées à J1, J3 et J6, patients immunocompétents (Ratio CD74 J3/J1 « élevé ») et patients immunodéprimés (Ratio CD74 J3/J1 « bas »).

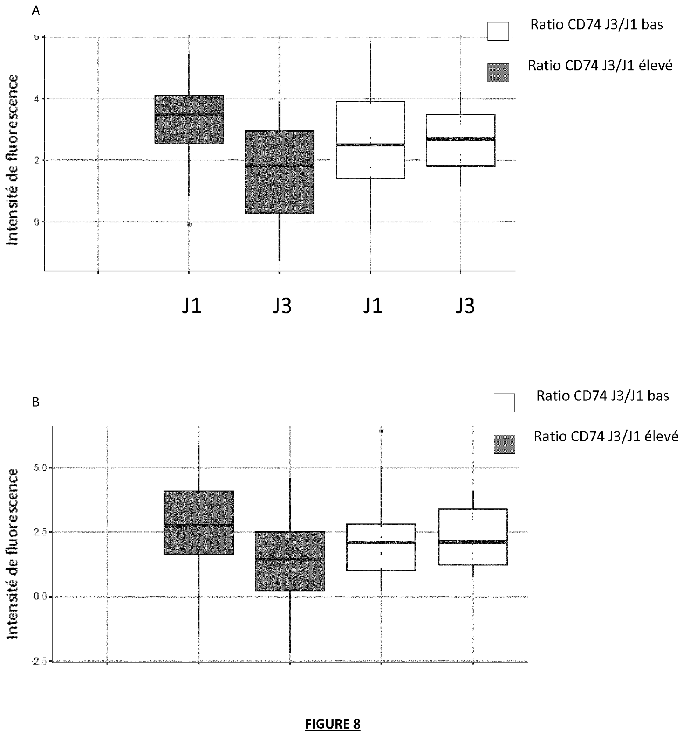

Figure 8 : La figure 8 montre l'expression des séquences SEQ ID NO 9 (A) et SEQ ID NO 10 (B), à partir de sang total de 102 patients en choc septique stratifiés en fonction du ratio entre le niveau d'expression de CD74 à J3 et le niveau d'expression de CD74 à J1 et quantifiées par RT-qPCR. L'axe des ordonnées représente les taux d'expression des séquences citées ci-dessus. Les conditions suivantes sont représentées à J1, J3 et J6, patients immunocompétents (Ratio CD74 J3/J1 « élevé ») et patients immunodéprimés (Ratio CD74 J3/J1 « bas »).

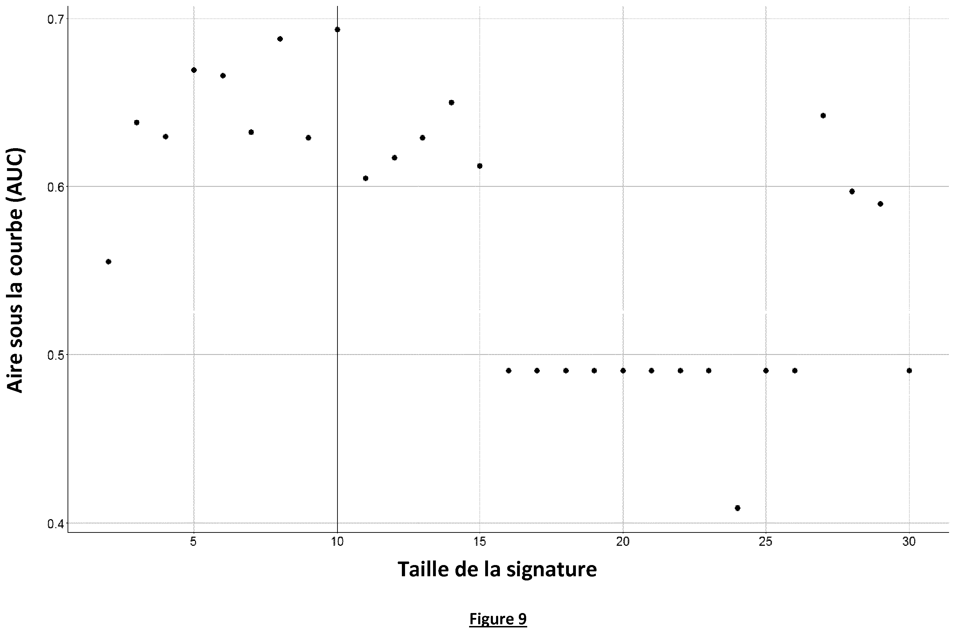

Figure 9 : La figure 9 montre un graphique représentant l'association entre la taille de la signature de marqueurs et le pouvoir discriminant entre les patients considérés comme immunocompétents et ceux considérés comme immunodéprimés.

La présente invention est illustrée de manière non limitative par les exemples suivants.

### Exemple 1 : Modèle de tolérance à l'endotoxine (ET)

La tolérance à l'endotoxine correspond à un état temporaire d'incapacité d'une cellule ou d'un organisme à répondre à une stimulation par une endotoxine, résultant d'une première stimulation par une endotoxine.

Le modèle de tolérance à l'endotoxine a été mis en place pour mimer d'une part un contexte inflammatoire induit par une stimulation par le lipopolysaccharide (LPS) et d'autre part l'anergie monocytaire qui représente un état de « non réponse » des cellules. Ces composantes de l'immunité sont retrouvées chez différents type patients, tels que les patients souffrant de sepsis, traumatisés, brûlés ou encore ayant subi une chirurgie lourde.

### Matériels et méthodes

### Stimulations des PBMC

Le modèle de tolérance à l'endotoxine a été mis en place à partir de 5 poches de sang citrate provenant de volontaires sains prélevés à l'EFS en accord avec les procédures standardisées pour le don de sang, et utilisés immédiatement après réception. Les PBMC, cellules mononuclées du sang périphériques, sont isolées grâce à un gradient de densité ajusté à 2 millions de cellules par mL et mises en culture dans du milieu X-Vivo (Lonza), à 37°C et 5 % de CO₂. L'endotoxine utilisée dans le cadre de ce modèle est le lipopolysaccharide (LPS), qui est un composant majeur de la membrane externe des bactéries Gram négatives. Le LPS est obtenu à partir d'un mélange de trois souches d'Escherichia coli : O111 :B4, O55 :B5 et 0127 :B8 (Sigma). L'ensemble des conditions sont réalisées en triplicats biologiques. Dans ce modèle *ex-vivo* de tolérance à l'endotoxine, les PBMC sont dans un premier temps mis en culture 15 heures sans (cellules contrôle NS et cellules LPS mimant la condition inflammatoire) ou avec une dose de 2 ng/mL de LPS (cellules ET mimant l'anergie monocytaire, condition d'immunosuppression). Après une étape de lavage, les PBMC sont incubées une seconde fois pendant 6 heures sans (cellules contrôle NS) ou avec une dose de 100 ng/mL de LPS (cellules LPS et ET).

A la fin des expériences, les surnageants de cultures sont récoltés et conservés à -80°C. Les cellules sont elles aussi récoltées, lysées et conservées à -80°C avant de réaliser l'extraction des ARN (kit commercial Qiagen) pour l'étude transcriptomique. Pour valider l'efficacité du modèle, les concentrations de la cytokine pro-inflammatoire TNF-α (« gène tolérisable ») et de la cytokine anti-inflammatoire IL10 (« gène non-tolérisable ») sont déterminées à partir des surnageants de culture de PBMC par ELISA (kits commerciaux R&D System).

### Extraction et amplification des ARN

Les ARNₘ sont extraits à l'aide de kits commerciaux (kit RNeasy Mini Plus, QIAGEN) à partir des cultures *ex vivo* de PBMC décrites ci-dessus.

Les ARNₘ totaux sont ensuite dosés et caractérisés. La caractérisation des ARNₘ est réalisée par électrophorèse capillaire à l'aide du Bioanalyser 2100. La qualité des échantillons d'ARNₘ est évaluée par le calcul du RIN (RNA Integrity Number). Cette valeur est basée sur la détection des ARN ribosomiques 18S et 28S, si le RIN tend vers 10 cela signifie que l'ARNₘ est intègre (un RIN ≥ à 7 est accepté).

La synthèse de l'ADN complémentaire (ADNc) et les étapes d'amplification sont réalisées par une méthode linéaire et isotherme décrite en 2005 par la société NuGEN Technologies (Kurn N et al. Novel isothermal, linear nucleic acid amplification systems for highly multiplexed applications. Clinical chemistry. 2005;51(10):1973-81). Le processus d'amplification Ribo-SPIA est utilisé (Watson JD et al. Complementary RNA amplification methods enhance microarray identification of transcripts expressed in the C. elegans nervous system. BMC genomics. 2008;9:84) à partir de 16 ng d'ARN total (kit commercial WTO pico, Nugen) et consiste en trois étapes. La première étape consiste en la production du premier brin d'ADN_{c} par une reverse transcription à partir d'une matrice ARNₘ, ceci par l'utilisation d'un mélange d'amorces aléatoires et oligo-dT. La seconde étape, consiste en l'ajout à la réaction de l'ADN polymérase, ce qui induit la production du second brin d'ADN_{c}. La troisième étape, engage l'amplification SPIA par déplacement de brin. Les amorces hybrides ADN/ARN sont dégradées par l'activité RNAse H de l'ADN polymérase lorsqu'elles sont complexées avec la matrice d'ADN_{c}. La synthèse d'ADN simple brin (complémentaire à la matrice ARNₘ) est amorcée et se poursuit, autorisant de nouvelles amorces SPIA à se fixer à la matrice d'ADN_{c}, entretenant ainsi le processus répétitif de synthèse de brin. Les ADN_{c} sont ensuite fragmentés en fragments de 50-200 pb à l'aide d'une DNase à partir de 5 µg d'ADN purifié et amplifié (kit commercial, Nugen) et sont également marqués en 3' (kit commercial Nugen). L'amplification et la fragmentation de l'ADN_{c} sont vérifiées sur Bioanalyser. Le profil d'amplification s'étend sur une gamme de tailles des ADN_{c} allant de 25 à 4 000 nucléotides, avec un pic aux alentours de 1 500 pb. Le profil de fragmentation doit quant à lui être centré sur une population d'acides nucléiques autour d'une taille de 100 nucléotides, ce qui est recommandé pour l'hybridation sur une puce à ADN Affymetrix.

### Analyse par Biopuce

L'identification de séquences présentant un différentiel d'expression repose sur la conception et l'utilisation d'une puce à ADN haute densité au format GeneChip, dénommée HERV-V3, conçue par les inventeurs et dont la fabrication a été sous-traitée à la société Affymetrix. Cette puce contient des sondes qui s'hybrident à des séquences HERV distinctes au sein du génome humain. Ces séquences sont extraites d'une base de donnée spécifique aux inventeurs, déjà publiée (Becker et al. A comprehensive hybridization model allows whole HERV transcriptome profiling using high density microarray, BMC Genomics 2017 18:286).

La puce HERV-V3 cible 353,994 éléments HERV/MaLR, et plus de 1500 gènes de l'immunité.

Une fois les ADN_{c} amplifiés et fragmentés, ils peuvent être hybridés sur la puce HERV-V3, au sein d'un four à 50°C pendant 18 heures avec une agitation constante à 60 rpm à hybridation. Un système de fluidique permet d'automatiser les étapes de lavages et de coloration, enfin après l'ensemble de ces étapes la puce est lue à l'aide d'un scanner fluorométrique.

Le jeu de données brut est créé à partir du regroupement des fichiers CEL de chaque puce par des méthodes classiques d'Affymetrix.

Après un premier contrôle qualité des données brutes, plusieurs étapes sont réalisées : correction du bruit de fond par la méthode Robust Multi-array Average (RMA), normalisation des données de chaque puce par les quantiles, regroupement des données de sondes en jeux de sondes (probes en probesets) et lissage de la médiane. Une seconde étape de contrôle qualité est réalisée. Toutes ces étapes permettent d'obtenir ainsi une matrice contenant les données normalisées.

Le prétraitement des puces ainsi que l'analyse statistique sont réalisés en utilisant R/Bioconductor.

Une étape préalable à l'analyse consiste à évaluer la qualité des puces, avant et après normalisation. Pour cela plusieurs critères sont à prendre en compte : la qualité de l'ARN, les contrôles d'amplification et de fragmentation de l'ADN_{c}, l'image des puces produites après le scan, l'hybridation des contrôles d'Affymetrix, l'intensité des signaux (avant et après normalisation), l'homogénéité des probesets (RLE et NUSE plots), la corrélation des puces (avant et après normalisation) et l'analyse en composante principale. Pour l'ensemble de ces critères, une analyse statistique permet d'identifier les valeurs extrêmes pour chaque puce et les données sont ensuite regroupées. Les puces qui passent moins de 5 contrôles qualité sont retirées de l'analyse. Pour chaque jeu de données, une table de décision est alors produite pour résumer l'ensemble des critères qualités, et identifier rapidement les puces à retirer de l'analyse.

Il est parfois nécessaire d'avoir recourt à une méthode corrective pour l'analyse des données. Cette correction dite COMBAT (pour Combining BATches) permet de corriger la variabilité technique du jeu de données et faire ainsi ressortir sa variabilité biologique.

Enfin, une étape de filtre des données a été réalisée pour réduire le jeu de données et gagner en puissance statistique pour les analyses. Le seuil d'intensité a été défini comme la valeur minimale d'intensité pour laquelle le 75^{ème} percentile de la distribution des coefficients de variation est en dessous de 10%. De cette manière, le seuil d'intensité est de 2^{5.5}. Les probesets sous le seuil d'intensité dans plus de 68 % de tous les échantillons (31 échantillons sur 45) sont éliminés.

Parmi les 71,063 probesets ciblant des HERVs/MaLRs et les 42,560 probesets ciblant des gènes sélectionnés dans l'étape précédente, une analyse d'expression différentielle a été réalisée.

Etudier l'expression différentielle entre deux conditions revient à calculer le ratio d'expression ou Fold Change (FC). Par exemple une valeur d'expression égale à 10 à la condition A, et une valeur égale à 5 sous la condition B, le FC de A/B est égal à 2. Les données de FC seront représentées en log2 FC. Pour déterminer qu'un gène ou qu'une séquence HERV/MaLR est différentiellement exprimé entre deux conditions, la méthode Limma a été utilisée (Smyth GK. Linear models and empirical bayes methods for assessing differential expression in microarray experiments. Statistical applications in genetics and molecular biology 2004, 3: Article3). Des tests statistiques et leur p-value associées sont calculées pour évaluer la significativité des changements d'expression observés. Les p-values ont été ajustées par un contrôle du taux de fausse découverte (FDR, en raison des tests multiples) selon la méthode de Benjamini et Hochberg (Hochberg et al. Controlling the False Discovery Rate: A Practical and Powerful Approach to Multiple Testing. Journal of the Royal Statistical Society Series B (Methodological) 1995, Vol. 57, No. 1 (1995), pp. 289-300). Un probeset est considéré comme étant significativement différentiellement exprimé lorsque la valeur absolue du FC en log2 est supérieure à 1 et que la p-value ajustée est en dessous de 0,05 (cf. figure 1).

Les séquences qui ont été identifiées comme étant les plus différentiellement exprimées par la biopuce ont été validées par RT-qPCR à partir des mêmes échantillons que ceux ayant permis la réalisation des puces HERV-V3.

### Résultats

Comme indiqué précédemment, pour valider l'efficacité du modèle les concentrations de la cytokine pro-inflammatoire TNF-α (« gène tolérisable ») et de la cytokine anti-inflammatoire IL10 (« gène non-tolérisable ») sont déterminées à partir des surnageants de culture de PBMC par ELISA (kits commerciaux R&D System).

Comme montré dans la figure 2A, les cellules stimulées deux fois par du LPS (modèle ET, condition d'immunosuppression) produisent de faibles quantités de TNF-α (100-500 pg/mL) comparées aux cellules stimulées uniquement une fois (modèle LPS, condition inflammatoire) (500-2000 pg/mL). En revanche, ces mêmes cellules sécrètent de plus fortes concentrations d'IL-10 (100-1000 pg/mL) en comparaison aux cellules stimulées une fois (50-400 pg/mL). Ces résultats au niveau protéique sont confirmés au niveau ARNₘ puisqu'une diminution significative de l'expression du TNF-α couplée à une augmentation de l'expression génique de l'IL-10 (figure 2B) dans les cellules stimulées deux fois par du LPS ont été observées en comparaison aux PBMC stimulés une seule fois.

Ces résultats permettent de valider l'efficacité de ce modèle.

Une analyse d'expression des HERV/MaIR et gènes dans les PBMC non stimulés (NS, contrôle), stimulés une fois au LPS (LPS, condition inflammatoire) ou deux fois stimulés au LPS (ET, condition d'immunosuppression) a été faite sur la biopuce HERV-V3, conçue par les inventeurs.

Le traitement des données générées par l'analyse des puces HERV-V3 à l'aide de cette méthode a permis d'identifier un ensemble de 4 jeux de sondes (ou « probesets »). Ces « probesets » sont les plus statistiquement différentiellement exprimées parmi les séquences présentant une différence d'expression statistiquement significative entre les différentes conditions (patients sains, condition inflammatoire et condition d'immunosuppression). Comme indiqué précédemment, pour que le différentiel d'expression soit statistiquement significatif il faut que la valeur absolue du log2 Fold Change soit supérieure ou égale à 1 et que la p-valeur ajustée soit inférieure ou égale à 0.05. Ces critères s'appliqueront pour tous les exemples. Ces 4 « probesets » sont associés à des séquences HERV identifiées par les SEQ ID NO 1 et 4 à 6. La localisation chromosomique de chaque séquence est donnée dans le référentiel GRCh38. Dans le

Tableau 9, ci-dessous, la liste des séquences identifiées.

**Tableau 9**

| **SEQ ID N°** | **NOM DU PROBESETS DE LA PUCE HERV-V3** | **NOM DE LA FAMILLE** | **LOCALISATION GRCH38 DE L'ELEMENT ENTIER** |
|---|---|---|---|
| SEQ ID NO 1 | 190665001-HERV0376 | LTR101 | chr19:54891074-54891496 |
| SEQ ID NO 4 | 121601801-HERV0492 | LTR33 | chr12:112971073-112971451 |
| SEQ ID NO 5 | 011052702-MALR1044 | MSTC | chr12:112971073-112971451 |
| SEQ ID NO 6 | 011052202-HERV1033 | MLT2B5 | chr1:78623489-78623954 |

Comme observé sur la figure 3C, l'expression de la séquence SEQ ID NO 1 est significativement, d'un point de vue statistique, plus importante dans la condition NS comparé aux conditions LPS et ET.

Les figures 3D et 3F illustrent le profil d'expression des séquences SEQ ID NO 4 et SEQ ID NO 5 observé dans les PBMC avec la puce HERV-V3. Ces figures montrent un profil d'expression similaire, à savoir, pour ces deux séquences, une augmentation de l'expression dans la condition LPS comparé aux conditions NS et ET, avec une différence statistiquement significative entre LPS et ET.

La séquence SEQ ID NO 6, quant à elle, a une expression plus importante dans les conditions NS et LPS comparé à la condition ET, avec des différences statistiquement significatives entre NS et ET, et entre LPS et ET (figure 3F).

Ces résultats montrent une modulation de l'expression de ces séquences dans le modèle d'endotoxine tolérance et ainsi leur capacité à être utilisées en tant que marqueur du statut immunitaire.

Les résultats montrent également que les séquences SEQ ID NO 4 à 6 possèdent un profil « tolérisable » (condition inflammatoire) dans un modèle de tolérance à l'endotoxine alors que la séquence SEQ ID NO 1 possède un profil « non-tolérisable » (condition d'immunosuppression) dans un modèle de tolérance à l'endotoxine.

La figure 3 illustre l'expression des séquences SEQ ID NO 1 (C), SEQ ID NO 4 (D), SEQ ID NO 5 (E), SEQ ID NO 6 (F), sur des PBMC, issues des mêmes 5 volontaires sains que pour la biopuce HERV-V3, stimulées par du LPS et quantifiées par RT-qPCR après réalisation du modèle de tolérance à l'endotoxine.

Les résultats montrent que le même profil que celui obtenu sur les biopuces est observé pour chaque séquence identifiée. Ainsi les données obtenues par la biopuce HERV-V3 sont confirmées par RT-qPCR (figure 3).

### Exemple 2 : Patients en soins intensifs

### Matériels et méthodes

### Patients et Echantillons biologiques

Cette étude observationnelle rétrospective a été conduite chez des patients de 37 à 77 ans (13 hommes, 7 femmes, âge médian: 59 ans) admis en soins intensifs suite à un choc septique.

Des échantillons de sang total ont été prélevés sur des tubes PAXgene (PreAnalytix) sur ces 20 patients en choc septique aux jours 1 (J1) et 3 ou 4 (J3) après l'admission en soins intensifs, puis ont été stockés (cohorte rétrospective).

Les patients de cette cohorte ont été stratifiés en fonction du niveau d'expression de HLA-DR à la surface des monocytes (mHLA-DR). L'expression de mHLA-DR a été mesurée à J3 (jours 3 ou 4) par cytométrie de flux.

20 patients de cette cohorte ont été sélectionnés, 10 patients (50%) avaient une expression élevée de HLA-DR au jour 3 ou 4 (plus de 30% d'expression), et 10 patients (50%) avaient une faible expression de HLA-DR au jour 3 ou 4 (moins de 30% d'expression).

Les patients ayant une expression élevée de HLA-DR sont considérés comme ayant un statut immunocompétent (DR+) et les patients ayant une expression faible de HLA-DR sont considérés comme ayant un statut immunodéprimé (DR-).
5 volontaires sains sont également inclus dans cette étude.

### Extraction des ARN

L'extraction de l'ARN a été effectuée à l'aide du kit PAXgene Blood RNA (PreAnalytix) en suivant les recommandations du fabricant. Avant l'étape d'élution de l'ARN, l'ADN génomique résiduel a été éliminé par l'action d'une DNAse. La concentration d'ARN a été déterminée par fluorimétrie (kit RNA assay sur Qubit, Life Technologies). La qualité de l'ARN a ensuite été contrôlée à l'aide du kit RNA 6000 Nano sur un Bioanalyseur (Agilent Technologies), les prélèvements avec un RIN (RNA Integrity Number) supérieur à 6 étant considérés de bonne qualité.

Les étapes d'amplification des ARN, d'analyse par biopuce et de validations des séquences par RT-qPCR qui ont été identifiées comme étant différentiellement exprimés par la biopuce sont mises en œuvre comme décrit dans l'exemple 1.

### Résultats

Le traitement des données générées par l'analyse des puces HERV-V3 à l'aide de cette méthode a permis d'identifier un ensemble de 6 jeux de sondes (ou « probesets »). Ces « probesets » sont les plus statistiquement différentiellement exprimées parmi les séquences présentant une différence d'expression statistiquement significative entre les deux conditions (condition immunocompétent et condition d'immunosuppression). Ces 6 « probesets » sont associés à des séquences HERV identifiées par les SEQ ID NO 2, 3, 7, 8, 11 et 12. La localisation chromosomique de chaque séquence est donnée dans le référentiel GRCh38. Dans le Tableau 10, ci-dessous, la liste des 6 séquences identifiées.

**Tableau 10**

| **SEQ ID N°** | **NOM DU PROBESET DE LA PUCE HERV-V3** | **NOM DE LA FAMILLE** | **LOCALISATION GRCH38 DE L'ELEMENT ENTIER** |
|---|---|---|---|
| SEQ ID NO 2 | 220247002-HERV0797 | MER4B | chr22:36153696-36154283 |
| SEQ ID NO 3 | 170369402HE41env | HERV-E4.1 | chr17:35505737-35508365 |
| SEQ ID NO 7 | 130360601-HERV0808 | MER50C | chr13:42884951-42886257 |
| SEQ ID NO 8 | 141107102-MALR1019 | MLT1J1 | chr14:91230494-91230820 |
| SEQ ID NO 11 | 050286701-HERV0513 | LTR40A | chr5:14551189-14551685 |
| SEQ ID NO 12 | 050287402-MALR1022 | MLT1K | chr5:14562791-14563322 |

Comme observé sur les figures 5A et 5C, l'expression des séquences SEQ ID NO 2 et 7 est diminuée chez les patients immunodéprimés (DR-) dès J1 après l'admission en soins intensifs comparé aux patients immunocompétents. Alors que l'expression des SEQ ID NO 3, 8, 11 et 12 est diminuée chez les patients immunodéprimés (DR-) à J3 après l'admission en soins intensifs comparé aux patients immunocompétents (cf. figures 5B, 5D, 5E et 5F).

Ainsi ces résultats montrent l'utilité des séquences SEQ ID NO 2 et 7 en tant que marqueurs, et ce dès J1, de l'immunosuppression. Les séquences SEQ ID NO 3, 8, 11 et 12, quant à elles, sont des marqueurs de l'immunosuppression à J3.

Ces séquences, identifiées comme étant différentiellement exprimées par la biopuce ont été validées par RT-qPCR. Les résultats sont illustrés à la figure 6.

Des profils similaires à ceux obtenus sur les biopuces sont observé pour chaque séquence identifiée. Ainsi les données obtenues par la biopuce HERV-V3 sont confirmées par RT-qPCR.

### Exemple 3 : Patients en réanimation

### Matériels et méthodes

### Patients et Echantillons biologiques

Cette étude observationnelle rétrospective a été conduite chez des patients admis en réanimation de 6 centres hospitaliers français de 2009 à 2011. Les critères d'inclusion étaient les suivants :
- patients âgés de 18 ans ou plus ;
- prédiction par le clinicien d'un durée de séjour en soins intensifs d'au moins 2 jours ;
- patients présentant au moins un site d'infection aiguë suspectée ou confirmée par le clinicien sur des manifestations cliniques ou paracliniques ;
- patients présentant au moins deux critères parmi les suivants:
   ∘ température supérieure à 38°C ou inférieure à 36°C ;
   ∘ rythme cardiaque supérieur à 90 battements par minute ;
   ∘ rythme respiratoire supérieur à 20 respirations par minute ou PaCO2< 32 mmHg ;
   ∘ nombre de leucocytes supérieur à 12000/mm3 ou inférieur à 4000/mm3.

Les critères d'exclusion étaient les suivants :
- une immunodépression préexistante, y compris une chimiothérapie récente ou un traitement immunosuppresseur,
- une dose élevée (> 5 mg / kg de prednisolone équivalente pendant une durée> 5 jours) ou un traitement prolongé (0,5 mg / kg de prednisolone> 30 jours) de corticostéroïdes;
- aplasie (neutrophiles circulants <500 cellules / mm3),
- immunodéficience primaire et
- circulation extracorporelle le mois précédant l'admission à l'unité de soins intensifs.

Parmi tous les patients, 102 patients remplissaient les critères suivants :
- patients en choc septique ;
- un premier prélèvement sanguin a été réalisé au maximum dans les 24 premières heures suivant l'arrivée du patient en soins intensifs (J1) ;
- un deuxième prélèvement sanguin a été réalisé entre 3 et 4 jours suivant l'arrivée du patient en soins intensifs (J3) ;
- un troisième prélèvement sanguin a été réalisé 6 jours suivant l'arrivée du patient en soins intensifs (J6).

Ensuite, les patients de cette cohorte ont été stratifiés en fonction du ratio du niveau d'expression de CD74 à J3 sur le niveau d'expression de CD74 à J1, réalisé en RT-qPCR. La cohorte a été divisée en 2 catégories. Les personnes ayant un ratio CD74 J3/J1 supérieur à 1,23 sont dits « élevées » et sont considérés comme ayant un statut immunocompétent. Ceux ayant un ratio CD74 J3/J1 inférieur à 1,23 sont dits « bas » et sont considérés comme ayant un statut immunodéprimé.

Parmi les 102 patients de cette cohorte, 52 patients (51%) sont considérés comme ayant un statut immunodéprimés (Ratio CD74 J3/J1 « bas ») et 50 patients (49%) sont considérés comme ayant un statut immunocompétent (Ratio CD74 J3/J1 « élevé »). L'étape d'extraction des ARN est mise en œuvre comme décrit dans l'exemple 2.

Les étapes d'amplification des ARN, d'analyse par biopuce et de validations par RT-qPCR (à J1 et J3) des séquences qui ont été identifiées comme étant différentiellement exprimées par la biopuce sont mises en œuvre comme décrit dans l'exemple 1.

### Résultats

Le traitement des données générées par l'analyse des puces HERV-V3 à l'aide de cette méthode a permis d'identifier un ensemble de 2 jeux de sondes (ou « probesets »). Ces « probesets » sont les plus statistiquement différentiellement exprimés parmi les séquences présentant une différence d'expression statistiquement significative entre les deux conditions (condition immunocompétent et condition d'immunosuppression). Ces 2 « probesets » sont associés à des séquences HERV identifiées par les SEQ ID NO 9 et 10. La localisation chromosomique de chaque séquence est donnée dans le référentiel GRCh38. Dans le Tableau 11, ci-dessous, les 2 séquences identifiées.

**Tableau 11**

| **SEQ ID N°** | **NOM DU PROBESET DE LA PUCE HERV-V3** | **NOM DE LA FAMILLE** | **LOCALISATION GRCH38 DE L'ELEMENT ENTIER** |
|---|---|---|---|
| SEQ ID NO 9 | 021460102-HERV0599uL | LTR82B | chr2:102363654-102366601 |
| SEQ ID NO 10 | 021456001-MALR1017uL | MLT1I | chr2:102013616-102013971 |

Comme observé sur la figure 7A, l'expression de la séquence SEQ ID NO 9 est plus élevée à J3 chez les patients ayant un ratio CD74 J3/J1 « bas » comparé aux patients ayant un ratio de CD74 J3/J1 « élevé ».

De manière identique pour l'expression de la séquence SEQ ID NO 10 (figure 7B), elle est plus élevée à J3 chez les patients ayant un ratio CD74 J3/J1 « bas » comparé aux patients ayant un ratio de CD74 J3/J1 « élevé ».

Ainsi ces résultats montrent l'utilité de ces séquences SEQ ID NO 9 et 10 en tant que marqueur de l'état d'immunosuppression à J3.

Ces séquences, identifiées comme étant différentiellement exprimées par la biopuce ont été validées par RT-qPCR à J1 et J3 à partir de 30 patients sur les 102 de la cohorte. Les résultats sont illustrés à la figure 8.

Des profils similaires à ceux obtenus sur les biopuces sont observés pour chaque séquence identifiée. Ainsi les données obtenues par la biopuce HERV-V3 sont confirmées par RT-qPCR à partir de 30 patients.

### Exemple 4 : Notation des séquences identifiées

Pour chacune des séquences identifiées dans les exemples de 1 à 3 (SEQ ID NO 1-12), les inventeurs ont attribué une note. Cette notation se base sur les profils d'expression, en puces, des séquences SEQ ID NO 1 à 12 observés sur la cohorte de l'exemple 2.

Les inventeurs ont attribué des notes allant de 1 à 4 étoiles. Tous les critères sont visuels, à partir des graphiques montrant l'expression des différentes séquences identifiées. Comme vu précédemment, toutes ces séquences ont déjà été sélectionnées sur la base du niveau d'expression et du différentielle d'expression. Le mode d'attribution des notes est décrit dans le Tableau 12, ci-dessous.

**Tableau 12**

| **CRITERES** | **NOTE** |
|---|---|
| Augmentation de l'expression chez les patients à DR bas à J3 et différence visible dès J1 | **** |
| Augmentation de l'expression chez les patients à DR bas à J3, différence non visible à J1 | *** |
| Diminution de l'expression chez les patients à DR bas dès J1, différence visible ou non à J3 | ** |
| Diminution de l'expression chez les patients à DR bas à J3, différence non visible à J1 | * |

Voici dans le Tableau 13, ci-dessous, la note obtenue pour chacune des séquences identifiées dans les exemples 1 à 3.

**Tableau 13**

| **SEQ ID N°** | **NOM DU PROBESET DE LA PUCE HERV-V3** | **LOCALISATION CHROMOSOMIQUE** | **SCORE** |
|---|---|---|---|
| SEQ ID 1 | 190665001-HERV0376 | chr19:54891074-54891496 | *** |
| SEQ ID 2 | 220247002-HERV0797 | chr22:36153696-36154283 | ** |
| SEQ ID 3 | 170369402HE41env | chr17:35505737-35508365 | * |
| SEQ ID 4 | 121601801-HERV0492 | chr12:112971073-112971451 | ** |
| SEQ ID 5 | 011052702-MALR1044 | chr1:78648318-78648697 | ** |
| SEQ ID 6 | 011052202-HERV1033 | chr1:78623489-78623954 | ** |
| SEQ ID 7 | 130360601-HERV0808 | chr13:42884951-42886257 | *** |
| SEQ ID 8 | 141107102-MALR1019 | chr14:91230494-91230820 | * |
| SEQ ID 9 | 021460102-HERV0599 | chr2:102363654-102366601 | *** |
| SEQ ID 10 | 021456001-MALR1017 | chr2:102013616-102013971 | *** |
| SEQ ID 11 | 050286701-HERV0513 | chr5:14551189-14551685 | **** |
| SEQ ID 12 | 050287402-MALR1022 | chr5:14562791-14563322 | **** |

### Exemple 5 : Marqueurs HERV de l'immunosuppression

Pour l'identification de marqueurs spécifiques de l'immunosuppression, les inventeurs ont utilisé les données générées par l'analyse des puces HERV-V3 à partir des échantillons de l'exemple 2.

Ainsi, les inventeurs ont sélectionné les séquences HERV différentiellement exprimées à J3 entre les patients considérés comme immunodéprimés (DR-) et ceux considérés comme immunocompétents (DR+), mais qui ne sont pas différentiellement exprimées entre les volontaires sains de l'étude et l'ensemble des patients (qu'il soit DR+ ou DR-, que ce soit à J1 ou à J3).

Cette sélection a permis d'identifier 17 séquences HERV. Pour chacune des séquences identifiées, les inventeurs ont attribué un score en fonction de plusieurs critères (profil d'expression, fold change, niveau d'expression, cohérence des profils d'expression entre les sondes sens et anti-sens, différence d'expression visible dès J1 et absence d'une trop grande variabilité entre patients). Ci-dessous le Tableau 14 décrit le mode d'attribution des points pour chaque critère.

**Tableau 14**

| **CRITERES** | **DESCRIPTION** | **POINTS** |
|---|---|---|
| **Profil d'expression** | Augmentation chez les patients considérés comme immunocompétents (DR -) par rapport à ceux considérés comme immunodéprimés (DR+) | 2 |
| | ou | ou |
| | Diminution chez patients considérés comme immunocompétents (DR -) par rapport à ceux considérés comme immunodéprimés (DR+) | 1 |
| **Niveau d'expression** | Niveau d'expression globalement au-dessus de 5 | 1 |
| Fold Change | Fold change élevé (log2 FC visuellement supérieur à 1.5) | 1 |
| Cohérence sens/antisens | Même profil d'expression entre les sondes sens et anti sens | 1 |
| Différence visible dès J1 | Différence entre patients DR haut et DR bas visible dès J1 (même si différence faible). | 1 |
| Variabilité | Malus si variabilité trop élevée. | -1 |
| **SCORE** | | **/6** |

Ainsi grâce à ce mode d'attribution d'un score, les inventeurs ont sélectionné 10 candidats et exclu 7 candidats dont les performances semblent insuffisantes. Voici dans le Tableau 15, ci-dessous, les 10 marqueurs identifiés avec leur score respectif.

**Tableau 15**

| **SEQ ID N°** | **NOM DU PROBESET DE LA PUCE HERV-V3** | **LOCALISATION CHROMOSOMIQUE** | **SCORE** |
|---|---|---|---|
| SEQ ID 13 | 052182701-MALR1129 | chr5:132453630-132454148 | 5 |
| SEQ ID 14 | 190478501-MALR1003 | chr19:41812466-41813010 | 4 |
| SEQ ID 15 | 011790601ERV9sLU5 | chr1:155637287-155637547 | 4 |
| SEQ ID 16 | 052681601-MALR1018 | chr5:170290289-170290812 | 4 |
| SEQ ID 17 | 160627301-MALR1014 | chr16:50662453-50662912 | 4 |
| SEQ ID 18 | 111686702-HERV0861 | chr11:122671887-122672147 | 3 |
| SEQ ID 8 | 141107102-MALR1019 | chr14:91230494-91230820 | 3 |
| SEQ ID 19 | 040318302-MALR1134 | chr4:15825146-15825565 | 2 |
| SEQ ID 20 | 041529101-MALR1026 | chr4:83464568-83464963 | 2 |
| SEQ ID 21 | 141106902-MALR1133 | chr14:91222760-91223118 | 2 |

Les séquences SEQ ID 13 à 18 et 19 à 21 sont des séquences nouvellement identifiées. Quant à la séquence SEQ ID 8, il s'agit d'une séquence qui a déjà été identifiée dans l'exemple 2.

### Exemple 6 : Marqueurs HERV de l'inflammation

Pour l'identification de marqueurs spécifiques de l'inflammation, les inventeurs ont utilisé les données générées par les analyses des puces HERV-V3 à partir des échantillons des exemples 1 et 2.

Ainsi, les inventeurs ont sélectionné les séquences HERV d'une part différentiellement exprimées entre la condition LPS (condition d'immunosuppression) et la condition NS (contrôles négatifs), dans l'exemple 1, et d'autre part différentiellement exprimées entre les patients à J1 ou J3 comparé aux volontaires sains, de l'exemple 2.

Cette sélection a permis d'identifier 13 séquences HERV. Comme dans l'exemple 5, pour chacune des séquences identifiées, les inventeurs ont attribué un score en fonction des mêmes critères. Le mode d'attribution des points pour chaque critère est décrit dans le Tableau 16, ci-dessous.

**Tableau 16**

| **CRITERES** | **DESCRIPTION** | **POINTS** |
|---|---|---|
| **Profil d'expression** | Augmentation chez patients par rapport aux volontaires sains | 2 |
| | ou | ou |
| | Diminution chez patients par rapport aux volontaires sains | 1 |
| **Niveau d'expression** | Niveau d'expression globalement au-dessus de 5 | 1 |
| **Fold Change** | Fold change élevé (log2 FC visuellement supérieur à 1.5) | 1 |
| **Cohérence sens/antisens** | Même profil d'expression entre les sondes sens et anti sens | 1 |
| **Différence visible dès J1** | Différence entre patients DR+ et DR- bas visible dès J1 (même si différence faible). | 1 |
| **Variabilité** | Malus si variabilité trop élevée. | -1 |
| **SCORE** | | **/6** |

**Grâce à ce mode d'attribution d'un score, les inventeurs ont sélectionné 7 candidats et exclu 6 candidats dont les performances semblent insuffisantes. Voici dans le**

Tableau 17, ci-dessous, les 7 marqueurs identifiés avec leur score respectif.

**Tableau 17**

| **SEQ ID N°** | **NOM DU PROBESET DE LA PUCE HERV-V3** | **LOCALISATION CHROMOSOMIQUE** | **SCORE** |
|---|---|---|---|
| SEQ ID 1 | 190665001-HERV0376 | chr19:54891074-54891496 | 6 |
| SEQ ID 1 | 190665002-HERV0376 | chr19:54891074-54891496 | 6 |
| SEQ ID 22 | 060281701-MALR1043 | chr6:18403673-18404108 | 5 |
| SEQ ID 23 | 043166601-MALR1018 | chr4:184850413-184850785 | 5 |
| SEQ ID 24 | 100090601-HERV0429 | chr10:5856198-5856795 | 4 |
| SEQ ID 25 | 061529601-HERV0492 | chr6:107800650-107801138 | 3 |
| SEQ ID 26 | 100871501-MALR1020 | chr10:60410534-60411224 | 3 |
| SEQ ID 27 | 170842002-MALR1003 | chr17:78345106-78345577 | 3 |

Cette stratégie d'identification de marqueur de l'inflammation a permis d'identifier 6 nouvelles séquences (SEQ ID 22 à 27) et de retrouver une séquence déjà identifiée dans l'exemple 1 (SEQ ID 1). A noter, également, que 2 jeux de sondes (probsets) ciblent la même séquence HERV (SEQ ID 1).

### Exemple 7 : Signature de marqueurs du statut immunitaire

Pour cet exemple, l'objectif était de déterminer une signature de marqueurs permettant de discriminer au mieux les patients considérés comme immunodéprimés et ceux considérés comme immunocompétents. Pour ce faire, les inventeurs ont utilisé les données générées par les analyses des puces HERV-V3 à partir des échantillons des exemples 2 et 3.

Les inventeurs ont sélectionné les séquences HERV qui étaient différentiellement exprimées à J3 entre les patients considérés comme immunodéprimés (DR-) et ceux considérés comme immunocompétents (DR+). Cette sélection a permis d'identifier une liste de 193 séquences HERV.

Afin d'obtenir une signature réduite permettant toujours de discriminer au mieux les patients considérés comme immunodéprimés et ceux considérés comme immunocompétents à partir de ces 193 séquences HERV, les inventeurs ont appliqué la méthode « Random Forests » (Tin Kam Ho, *Random Decision Forests,* AT&T Bell Laboratories). Cette méthode permet de classer chaque séquence en fonction de son pouvoir discriminant entre les deux conditions étudiées (immunodéprimés et immunocompétents).

Ensuite, pour connaitre le nombre optimal de séquences devant composer la signature, les inventeurs ont calculé les performances de prédiction sur la cohorte de l'exemple 3, en utilisant le pouvoir discriminant pour des signatures de taille allant de 2 à 30 marqueurs entre les patients ayant un ratio de CD74 entre J3 et J1 bas et ceux avec ce même ratio élevé. Ainsi ils ont déterminé que la signature d'une taille de 10 séquences avait les meilleures performances (meilleure aire sous la courbe : AUC) (cf. figure 9). Dans le Tableau 18, ci-dessous, la liste des marqueurs composant la signature permettant de discriminer au mieux les patients considérés comme immunodéprimés et les patients considérés comme immunocompétents.

**Tableau 18**

| **SEQ ID N°** | **NOM DU PROBESET DE LA PUCE HERV-V3** | **LOCALISATION CHROMOSOMIQUE** |
|---|---|---|
| SEQ ID 28 | 081921103-HERV0958 | chr8:125945973-125951030 |
| SEQ ID 29 | 032622601MR41sLU5p | chr3:167401329-167401866 |
| SEQ ID 30 | 220246901-HERV0889 | chr22:36147793-36148208 |
| SEQ ID 31 | 061827101-HERV0856 | chr6:127790579-127792191 |
| SEQ ID 3 | 170369402HE41env | chr17:35505737-35508365 |
| SEQ ID 32 | 170828901-HERV0770 | chr17:77462942-77463350 |
| SEQ ID 28 | 081921101-HERV0958 | chr8:125945973-125951030 |
| SEQ ID 28 | 081921102-HERV0958 | chr8:125945973-125951030 |
| SEQ ID 33 | 190148802-MALR1127 | chr19:14612123-14612747 |
| SEQ ID 34 | 120093401-HERV1034 | chr12:9038254-9038598 |

A noter que cette signature est composée de 8 séquences utilisant 10 jeux de sondes différents. En effet, 3 jeux de sondes ciblent la même séquence (SEQ ID NO 28). De plus dans cette signature nous retrouvons la séquence SEQ ID 3 déjà identifiée dans l'exemple 2. Les séquences de HERV apparaissent dans le tableau ci-dessus selon un ordre reflétant leur « importance » dans la stratification des patients selon leur statut immunitaire, en fonction d'un score qui leur a été attribué par l'algorithme de classification Random forests.

## Revendications

1. Procédé pour déterminer *in vitro* ou *ex vivo* le statut immunitaire d'un individu, dans lequel l'individu est un patient atteint de traumatismes, un patient atteint de brûlures, un patient ayant reçu une chirurgie ou un patient en état septique, de préférence un patient atteint de choc septique, ledit procédé comprenant une étape de détection et/ou de quantification de l'expression, dans un échantillon biologique test dudit individu, appelé aussi échantillon biologique test, d'au moins une partie d'au moins une séquence de HERV/MaLR choisie parmi les séquences identifiées en SEQ ID NOs : 1 à 34 ou parmi les séquences qui présentent au moins 99% d'identité avec une des séquences identifiées en SEQ ID NOs : 1 à 34, parmi les listes suivantes :
- Liste 1 :
| **SEQ ID NO** | **Localisation GRCh38** | **Nom du probeset correspondant de la puce HERV-V3** |
|---|---|---|
| 1 | chr19:54891074-54891496 | 190665001-HERV0376 |
| 2 | chr22:36153696-36154283 | 220247002-HERV0797 |
| 3 | chr17:35505737-35508365 | 170369402HE41env |
| 4 | chr12:112971073-112971451 | 121601801-HERV0492 |
| 5 | chr1:78648318-78648697 | 011052702-MALR1044 |
| 6 | chr1:78623489-78623954 | 011052202-HERV1033 |
| 7 | chr13:42884951-42886257 | 130360601-HERV0808 |
| 8 | chr14:91230494-91230820 | 141107102-MALR1019 |
| 9 | chr2:102363654-102366601 | 021460102-HERV0599uL |
| 10 | chr2:102013616-102013971 | 021456001-MALR1017uL |
| 11 | chr5:14551189-14551685 | 050286701-HERV0513 |
| 12 | chr5:14562791-14563322 | 050287402-MALR1022 |
- Liste 2 :
| **SEQ ID NO** | **Localisation GRCh38** | **Nom du probeset correspondant de la puce HERV-V3** |
|---|---|---|
| 13 | chr5:132453630-132454148 | 052182701-MALR1129 |
| 14 | chr19:41812466-41813010 | 190478501-MALR1003 |
| 15 | chr1:155637287-155637547 | 011790601ERV9sLU5 |
| 16 | chr5:170290289-170290812 | 052681601-MALR1018 |
| 17 | chr16:50662453-50662912 | 160627301-MALR1014 |
| 18 | chr11:122671887-122672147 | 111686702-HERV0861 |
| 8 | chr14:91230494-91230820 | 141107102-MALR1019 |
| 19 | chr4:15825146-15825565 | 040318302-MALR1134 |
| 20 | chr4:83464568-83464963 | 041529101-MALR1026 |
| 21 | chr14:91222760-91223118 | 141106902-MALR1133 |
- Liste 3 :
| **SEQ ID NO** | **Localisation GRCh38** | **Nom du probeset correspondant de la puce HERV-V3** |
|---|---|---|
| 1 | chr19:54891074-54891496 | 190665001-HERV0376 |
| 1 | chr19:54891074-54891496 | 190665002-HERV0376 |
| 22 | chr6:18403673-18404108 | 060281701-MALR1043 |
| 23 | chr4:184850413-184850785 | 043166601-MALR1018 |
| 24 | chr10:5856198-5856795 | 100090601-HERV0429 |
| 25 | chr6:107800650-107801138 | 061529601-HERV0492 |
| 26 | chr10:60410534-60411224 | 100871501-MALR1020 |
| 27 | chr17:78345106-78345577 | 170842002-MALR1003 |
- Liste 4 :
| **SEQ ID NO** | **Localisation GRCh38** | **Nom du probeset correspondant de la puce HERV-V3** |
|---|---|---|
| 28 | chr8:125945973-125951030 | 081921103-HERV0958 |
| 29 | chr3:167401329-167401866 | 032622601MR41sLU5p |
| 30 | chr22:36147793-36148208 | 220246901-HERV0889 |
| 31 | chr6:127790579-127792191 | 061827101-HERV0856 |
| 3 | chr17:35505737-35508365 | 170369402HE41env |
| 32 | chr17:77462942-77463350 | 170828901-HERV0770 |
| 28 | chr8:125945973-125951030 | 081921101-HERV0958 |
| 28 | chr8:125945973-125951030 | 081921102-HERV0958 |
| 33 | chr19:14612123-14612747 | 190148802-MALR1127 |
| 34 | chr12:9038254-9038598 | 120093401-HERV1034 |

2. Procédé selon la revendication 1, comprenant en outre les étapes suivantes :
- on compare l'expression dans l'échantillon biologique test, avec une expression de référence, ou avec l'expression dans un échantillon biologique de référence,
- puis on détermine le statut immunitaire dudit individu à partir de ladite comparaison.

3. Procédé selon l'une des revendications 1 et 2, dans lequel l'expression est détectée et/ou quantifiée au niveau transcrit ARN ou ARNm.

4. Procédé selon la revendication 3, dans lequel l'expression est détectée et/ou quantifiée par une méthode d'hybridation, de préférence avec une puce d'hybridation, par hybridation *in situ* ou par Northern blot, par une méthode d'amplification, de préférence par RT-qPCR, ou par séquençage, de préférence par séquençage haut débit.

5. Procédé selon l'une des revendications 1 à 4, dans lequel le statut immunitaire est déterminé comme étant un statut d'immunosuppression, un statut d'immunocompétence, ou un statut d'inflammation.

6. Procédé selon l'une des revendications 1 à 5, dans lequel l'échantillon biologique test provient d'un prélèvement qui a été obtenu dans les 10 jours suivant l'admission au sein de la structure médicale.

7. Procédé selon l'une des revendications 2 à 6, dans lequel l'échantillon biologique de référence est un échantillon biologique issu d'un individu sain, de préférence un échantillon biologique issu du même individu dont est issu l'échantillon biologique test mais prélevé avant l'infection ou l'agression, ou un échantillon biologique d'un individu de statut immunitaire connu, de préférence avec un statut d'inflammation, un statut immunitaire normal ou un statut d'immunosuppression.

8. Utilisation d'un kit comprenant des moyens d'amplification et/ou de détection d'au moins une séquence choisie parmi les séquences identifiées en SEQ ID NOs : 1 à 34 ou parmi les séquences qui présentent au moins 99% d'identité avec une des séquences identifiées en SEQ ID NOs : 1 à 34, **caractérisé en ce que** lesdits moyens d'amplification et/ou de détection permettent l'amplification et/ou la détection d'au plus 100 biomarqueurs, au total, pour déterminer *in vitro* ou *ex vivo* le statut immunitaire d'un individu, dans lequel l'individu est un patient atteint de traumatismes, un patient atteint de brûlures, un patient ayant reçu une chirurgie ou un patient en état septique, de préférence un patient atteint de choc septique.

9. Utilisation selon la revendication 8, dans lequel :
- les moyens d'amplification comprennent au moins un couple d'amorces consistant en deux amorces d'amplification choisies parmi des amorces d'amplification comprenant une séquence nucléotidique complémentaire d'au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences, ledit au moins un couple d'amorces d'amplification permettant d'amplifier, de préférence permettant d'amplifier spécifiquement, au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences, et/ou
- les moyens de détection comprennent au moins une sonde d'hybridation dont la séquence nucléotidique comprend une séquence nucléotidique complémentaire d'au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences.

10. Utilisation selon la revendication 8 ou 9, **caractérisé en ce que** l'au moins un couple d'amorces d'amplification est choisi parmi les couples d'amorces d'amplification suivants :
| **Couple d'amorces #** | **Amorces # (SEQ ID NOs, séquence nucléotidique)** |
|---|---|
| 1 | Forward : Amorce #1A (SEQ ID No : 35, TGTACAAAACTCAAATGGTCTTC) |
| | Reverse : Amorce #1B (SEQ ID No : 36, ATGACCAACTTAGATTTCCTTGA) |
| 2 | Forward : Amorce #2A (SEQ ID No : 37, GCCAGAGAGGCATAATGAAGCA) |
| | Reverse : Amorce #2B (SEQ ID No : 38, GATTCTAAGCCTCCCCCTCATTT) |
| 3 | Forward : Amorce #3A (SEQ ID No : 39, TGGCTCATAGGGATTCCAGACT) |
| | Reverse : Amorce #3B (SEQ ID No : 40, AGCAAGTTGTCAAGAGCCAATCT) |
| 4 | Forward : Amorce #4A (SEQ ID No : 41, CACTCTAGGAATCTTAGGCA) |
| | Reverse : Amorce #4B (SEQ ID No : 42, TGAAACCAATAGTCCAGTG) |
| 5 | Forward : Amorce #5A (SEQ ID No : 43, TTCTACTGTTCACTGCTATCCTCC) |
| | Reverse : Amorce #5B (SEQ ID No : 44, CCTGTGGCAGCTTTTTGAAGTAA) |
| 6 | Forward : Amorce #6A (SEQ ID No : 45, AGAGCAGAAGAAGATGGATACT) |
| | Reverse : Amorce #6B (SEQ ID No : 46, CATGAGCTGACATCATCCAAT) |
| 7 | Forward : Amorce #7A (SEQ ID No : 47, TCTGTACTGGTTGCCCCAAC) |
| | Reverse : Amorce #7B (SEQ ID No : 48, CGTGCCAGGCCTCTAATACTTTT) |
| 8 | Forward : Amorce #8A (SEQ ID No : 49, AGGGAAGACCCCAAGATGATG) |
| | Reverse : Amorce #8B (SEQ ID No : 50, CATGCAAAGTCCAACGAGAGG) |
| 9 | Forward : Amorce #9A (SEQ ID No : 51, GGGTGGCTGCATCCTATGG) |
| | Reverse : Amorce #9B (SEQ ID No : 52, CTGGTCAGGAAAAAATTTGCCTTC) |
| 10 | Forward : Amorce #10A (SEQ ID No : 53, ACATGACATTGTCTGAACTTTGGG) |
| | Reverse : Amorce #10B (SEQ ID No : 54, TAGGACCATGCAGATACTAGTGAC) |
| 11 | Forward : Amorce #11A (SEQ ID No : 55, GAACTCCACAAACCTTGA) |
| | Reverse : Amorce #11B (SEQ ID No : 56, GCTAGAAGCTTTGGATATCT) |
| 12 | Forward : Amorce #12A (SEQ ID No : 57, TGGCTGTTACAACTTTCATG) |
| | Reverse : Amorce #12B (SEQ ID No : 58, TCTCCCTATTCTGAGCACA) |

11. Utilisation selon l'une des revendications 8 à 10, **caractérisé en ce que** l'au moins une sonde d'hybridation est choisie parmi les sondes d'hybridation de séquences SEQ ID NO : 59 à 274.

12. Utilisation selon l'une des revendications 8 à 11, comprenant en outre des moyens d'amplification et/ou de détection d'autres biomarqueurs, en particulier des biomarqueurs endogènes, tels que d'autres HERV/MaLR et/ou des gènes, de préférence des gènes impliqués dans l'inflammation et/ou l'immunité, et/ou des gènes de ménage, et/ou des biomarqueurs exogènes, tels que des virus.

13. Utilisation :
- d'au moins une séquence choisie parmi les séquences identifiées en SEQ ID NOs : 1 à 34 ou parmi les séquences qui présentent au moins 99% d'identité avec une des séquences identifiées en SEQ ID NOs : 1 à 34 ; et/ou
- d'au moins un couple d'amorces d'amplification, consistant en deux amorces d'amplification choisies parmi des amorces d'amplification comprenant une séquence nucléotidique complémentaire d'au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences, ledit au moins un couple d'amorces d'amplification permettant d'amplifier, de préférence permettant d'amplifier spécifiquement, au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences ; et/ou
- d'au moins une sonde d'hybridation dont la séquence nucléotidique comprend une séquence nucléotidique complémentaire d'au moins une partie d'une séquence choisie parmi les séquences identifiées en SEQ ID NOs : 1 à 34 ou parmi les séquences qui présentent au moins 99% d'identité avec une des séquences identifiées en SEQ ID NOs : 1 à 34, et les séquences complémentaires de ces séquences, pour déterminer in vitro ou ex vivo le statut immunitaire d'un individu, dans lequel l'individu est un patient atteint de traumatismes, un patient atteint de brûlures, un patient ayant reçu une chirurgie ou un patient en état septique, de préférence un patient atteint de choc septique.

14. Utilisation selon la revendication 13, **caractérisée en ce que** :
- l'au moins un couple d'amorces d'amplification est choisi parmi les couples d'amorces d'amplification #1 à 12 selon la revendication 10; et/ou
- l'au moins une sonde d'hybridation est choisie parmi les sondes d'hybridation de séquences SEQ ID NOs : 59 à 274.

## Patentansprüche

1. Verfahren zur Bestimmung des Immunstatus eines Individuums *in vitro* oder *ex vivo,* wobei es sich bei dem Individuum um einen Patienten mit Verletzungen, einen Patienten mit Verbrennungen, einen mittels Chirurgie operierten Patienten oder einen Patienten mit einer Sepsis, vorzugsweise einen Patienten mit einem septischen Schock, handelt, wobei das Verfahren einen Schritt des Nachweisens und/oder der Quantifizierung der Expression, in einer biologischen Testprobe des Individuums, auch biologische Testprobe genannt, von mindestens einem Teil mindestens einer Sequenz von HERV/MaLR umfasst, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34, aus den folgenden Listen:
- Liste 1 :
| SEQ ID NO | Lokalisierung GRCh38 | Name des Sondensatzes, entsprechend dem Mikroarray HERV-V3 |
|---|---|---|
| 1 | chr19:54891074-54891496 | 190665001-HERV0376 |
| 2 | chr22:36153696-36154283 | 220247002-HERV0797 |
| 3 | chr17:35505737-35508365 | 170369402HE41env |
| 4 | chr12:112971073-112971451 | 121601801-HERV0492 |
| 5 | chr1:78648318-78648697 | 011052702-MALR1044 |
| 6 | chr1:78623489-78623954 | 011052202-HERV1033 |
| 7 | chr13:42884951-42886257 | 130360601-HERV0808 |
| 8 | chr14:91230494-91230820 | 141107102-MALR1019 |
| 9 | chr2:102363654-102366601 | 021460102-HERV0599uL |
| 10 | chr2:102013616-102013971 | 021456001-MALR1017uL |
| 11 | chr5:14551189-14551685 | 050286701-HERV0513 |
| 12 | chr5:14562791-14563322 | 050287402-MALR1022 |
- Liste 2 :
| SEQ ID NO | Lokalisierung GRCh38 | Name des Sondensatzes, entsprechend dem Mikroarray HERV-V3 |
|---|---|---|
| 13 | chr5:132453630-132454148 | 052182701-MALR1129 |
| 14 | chr19:41812466-41813010 | 190478501-MALR1003 |
| 15 | chr1:155637287-155637547 | 011790601ERV9sLU5 |
| 16 | chr5:170290289-170290812 | 052681601-MALR1018 |
| 17 | chr16:50662453-50662912 | 160627301-MALR1014 |
| 18 | chr11:122671887-122672147 | 111686702-HERV0861 |
| 8 | chr14:91230494-91230820 | 141107102-MALR1019 |
| 19 | chr4:15825146-15825565 | 040318302-MALR1134 |
| 20 | chr4:83464568-83464963 | 041529101-MALR1026 |
| 21 | chr14:91222760-91223118 | 141106902-MALR1133 |
- Liste 3 :
| SEQ ID NO | Lokalisierung GRCh38 | Name des Sondensatzes, entsprechend dem Mikroarray HERV-V3 |
|---|---|---|
| 1 | chr19:54891074-54891496 | 190665001-HERV0376 |
| 1 | chr19:54891074-54891496 | 190665002-HERV0376 |
| 22 | chr6:18403673-18404108 | 060281701-MALR1043 |
| 23 | chr4:184850413-184850785 | 043166601-MALR1018 |
| 24 | chr10:5856198-5856795 | 100090601-HERV0429 |
| 25 | chr6:107800650-107801138 | 061529601-HERV0492 |
| 26 | chr10:60410534-60411224 | 100871501-MALR1020 |
| 27 | chr17:78345106-78345577 | 170842002-MALR1003 |
- Liste 4 :
| SEQ ID NO | Lokalisierung GRCh38 | Name des Sondensatzes, entsprechend dem Mikroarray HERV-V3 |
|---|---|---|
| 28 | chr8:125945973-125951030 | 081921103-HERV0958 |
| 29 | chr3:167401329-167401866 | 032622601MR41sLU5p |
| 30 | chr22:36147793-36148208 | 220246901-HERV0889 |
| 31 | chr6:127790579-127792191 | 061827101-HERV0856 |
| 3 | chr17:35505737-35508365 | 170369402HE41env |
| 32 | chr17:77462942-77463350 | 170828901-HERV0770 |
| 28 | chr8:125945973-125951030 | 081921101-HERV0958 |
| 28 | chr8:125945973-125951030 | 081921102-HERV0958 |
| 33 | chr19:14612123-14612747 | 190148802-MALR1127 |
| 34 | chr12:9038254-9038598 | 120093401-HERV1034 |

2. Verfahren nach Anspruch 1, ferner umfassend die folgenden Schritte:
- Vergleich der Expression in der biologischen Testprobe mit einer Referenzexpression oder mit der Expression in einer biologischen Referenzprobe,
- sodann Bestimmung des Immunstatus des Individuums auf Basis des Vergleichs.

3. Verfahren nach einem der Ansprüche 1 und 2, wobei die Expression auf RNA- oder mRNA-Transkript-Ebene nachgewiesen und/oder quantifiziert wird.

4. Verfahren nach Anspruch 3, wobei die Expression mittels eines Hybridisierungsverfahrens, bevorzugt mit einem Hybridisierungs-Mikroarray, mittels Hybridisierung *in situ* oder mittels Northern-Blot, mittels eines Amplifikationsverfahrens, bevorzugt mittels RT-qPCR, oder mittels Sequenzierung, bevorzugt mittels Hochdurchsatzsequenzierung, nachgewiesen und/oder quantifiziert wird.

5. Verfahren nach einem der Ansprüche 1 bis 4, wobei der Immunstatus bestimmt wird als ein Status der Immunsuppression, ein Status der Immunkompetenz oder ein Status der Entzündung.

6. Verfahren nach einem der Ansprüche 1 bis 5, wobei die biologische Testprobe aus einer Probenahme stammt, die in den 10 Tagen ab der Aufnahme in eine medizinische Einrichtung erhalten wurde.

7. Verfahren nach einem der Ansprüche 2 bis 6, wobei es sich bei der biologischen Referenzprobe um eine biologische Probe, gewonnen aus einem gesunden Individuum, vorzugsweise um eine biologische Probe, gewonnen aus demselben Individuum, aus dem die biologische Testprobe gewonnen wird, aber entnommen vor der Infektion oder Störung, oder um eine biologische Probe eines Individuums mit bekanntem Immunstatus, vorzugsweise mit einem Status der Entzündung, einem normalen Immunstatus oder einem Status der Immunsuppression, handelt.

8. Verwendung eines Kits, umfassend Mittel zur Amplifikation und/oder zum Nachweisen mindestens einer Sequenz, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34, **dadurch gekennzeichnet, dass** die Mittel zur Amplifikation und/oder zum Nachweisen die Amplifikation und/oder den Nachweis von höchstens 100 Biomarkern insgesamt zur Bestimmung des Immunstatus eines Individuums *in vitro* oder *ex vivo* ermöglichen, wobei es sich bei dem Individuum um einen Patienten mit Verletzungen, einen Patienten mit Verbrennungen, einen mittels Chirurgie operierten Patienten oder einen Patienten mit einer Sepsis, vorzugsweise einen Patienten mit einem septischen Schock, handelt.

9. Verwendung nach Anspruch 8, wobei:
- die Mittel zur Amplifikation mindestens ein Primerpaar umfassen, bestehend aus zwei Amplifikationsprimern, ausgewählt aus Amplifikationsprimern, umfassend eine Nukleotidsequenz, die komplementär ist zu mindestens einem Teil einer Sequenz, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34, und den zu diesen Sequenzen komplementären Sequenzen, wobei das mindestens eine Amplifikationsprimerpaar das Amplifizieren, vorzugsweise das spezifische Amplifizieren von mindestens einem Teil einer Sequenz ermöglicht, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34, und den zu diesen Sequenzen komplementären Sequenzen, und/oder
- die Mittel zum Nachweisen mindestens eine Hybridisierungssonde umfassen, deren Nukleotidsequenz eine Nukleotidsequenz umfasst, die komplementär ist zu mindestens einem Teil einer Sequenz, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34, und den zu diesen Sequenzen komplementären Sequenzen.

10. Verwendung nach Anspruch 8 oder 9, **dadurch gekennzeichnet, dass** das mindestens eine Amplifikationsprimerpaar ausgewählt ist aus den folgenden Amplifikationsprimerpaaren:
| Primerpaar # | Primer # (SEQ ID NOs, Nukleotidsequenz) |
|---|---|
| 1 | Vorwärts : Primer #1A (SEQ ID No : 35, TGTACAAAACTCAAATGGTCTTC) |
| | Rückwärts : Primer #1B (SEQ ID No : 36, ATGACCAACTTAGATTTCCTTGA) |
| 2 | Vorwärts : Primer #2A (SEQ ID No : 37, GCCAGAGAGGCATAATGAAGCA) |
| | Rückwärts : Primer #2B (SEQ ID No : 38, GATTCTAAGCCTCCCCCTCATTT) |
| 3 | Vorwärts : Primer #3A (SEQ ID No : 39, TGGCTCATAGGGATTCCAGACT) |
| | Rückwärts : Primer #3B (SEQ ID No : 40, AGCAAGTTGTCAAGAGCCAATCT) |
| 4 | Vorwärts : Primer #4A (SEQ ID No : 41, CACTCTAGGAATCTTAGGCA) |
| | Rückwärts : Primer #4B (SEQ ID No : 42, TGAAACCAATAGTCCAGTG) |
| 5 | Vorwärts : Primer #5A (SEQ ID No : 43, TTCTACTGTTCACTGCTATCCTCC) |
| | Rückwärts : Primer #5B (SEQ ID No : 44, CCTGTGGCAGCTTTTTGAAGTAA) |
| 6 | Vorwärts : Primer #6A (SEQ ID No : 45, AGAGCAGAAGAAGATGGATACT) |
| | Rückwärts : Primer #6B (SEQ ID No : 46, CATGAGCTGACATCATCCAAT) |
| 7 | Vorwärts : Primer #7A (SEQ ID No : 47, TCTGTACTGGTTGCCCCAAC) |
| | Rückwärts : Primer #7B (SEQ ID No : 48, CGTGCCAGGCCTCTAATACTTTT) |
| 8 | Vorwärts : Primer #8A (SEQ ID No : 49, AGGGAAGACCCCAAGATGATG) |
| | Rückwärts : Primer #8B (SEQ ID No : 50, CATGCAAAGTCCAACGAGAGG) |
| 9 | Vorwärts : Primer #9A (SEQ ID No : 51, GGGTGGCTGCATCCTATGG) |
| | Rückwärts : Primer #9B (SEQ ID No : 52, CTGGTCAGGAAAAAATTTGCCTTC) |
| 10 | Vorwärts : Primer #10A (SEQ ID No : 53, ACATGACATTGTCTGAACTTTGGG) |
| | Rückwärts : Primer #10B (SEQ ID No : 54, TAGGACCATGCAGATACTAGTGAC) |
| 11 | Vorwärts : Primer #11A (SEQ ID No : 55, GAACTCCACAAACCTTGA) |
| | Rückwärts : Primer #11B (SEQ ID No : 56, GCTAGAAGCTTTGGATATCT) |
| 12 | Vorwärts : Primer #12A (SEQ ID No : 57, TGGCTGTTACAACTTTCATG) |
| | Rückwärts : Primer #12B (SEQ ID No : 58, TCTCCCTATTCTGAGCACA) |

11. Verwendung nach einem der Ansprüche 8 bis 10, **dadurch gekennzeichnet, dass** die mindestens eine Hybridisierungssonde ausgewählt ist aus den Hybridisierungssonden der Sequenzen SEQ ID NO : 59 bis 274.

12. Verwendung nach einem der Ansprüche 8 bis 11, ferner umfassend Mittel zur Amplifikation und/oder zum Nachweisen von anderen Biomarkern, insbesondere endogenen Biomarkern, wie anderen HERV/MaLR, und/oder Genen, vorzugsweise an Entzündung und/oder Immunität beteiligten Genen, und/oder Haushaltsgenen und/oder exogenen Biomarkern, wie Viren.

13. Verwendung:
- von mindestens einer Sequenz, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34; und/oder
- von mindestens einem Amplifikationsprimerpaar, bestehend aus zwei Amplifikationsprimern, ausgewählt aus Amplifikationsprimern, umfassend eine Nukleotidsequenz, die komplementär ist zu mindestens einem Teil einer Sequenz, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34, und den zu diesen Sequenzen komplementären Sequenzen, wobei das mindestens eine Amplifikationsprimerpaar das Amplifizieren, vorzugsweise das spezifische Amplifizieren von mindestens einem Teil einer Sequenz ermöglicht, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34, und den zu diesen Sequenzen komplementären Sequenzen; und/oder
- von mindestens einer Hybridisierungssonde, deren Nukleotidsequenz eine Nukleotidsequenz umfasst, die komplementär ist zu mindestens einem Teil einer Sequenz, ausgewählt aus den Sequenzen, identifiziert in den SEQ ID NOs : 1 bis 34, oder aus den Sequenzen, aufweisend mindestens 99 % Identität mit einer der Sequenzen, identifiziert in den SEQ ID NOs : 1 bis 34, und den zu diesen Sequenzen komplementären Sequenzen,
zur Bestimmung des Immunstatus eines Individuums *in vitro* oder *ex vivo,* wobei es sich bei dem Individuum um einen Patienten mit Verletzungen, einen Patienten mit Verbrennungen, einen mittels Chirurgie operierten Patienten oder einen Patienten mit einer Sepsis, vorzugsweise einen Patienten mit einem septischen Schock, handelt.

14. Verwendung nach Anspruch 13, **dadurch gekennzeichnet, dass** :
- das mindestens eine Amplifikationsprimerpaar ausgewählt ist aus den Amplifikationsprimerpaaren #1 bis 12 nach Anspruch 10;
und/oder
- die mindestens eine Hybridisierungssonde ausgewählt ist aus den Hybridisierungssonden der Sequenzen SEQ ID NOs : 59 bis 274.

## Claims

1. A Method for determining in vitro or ex vivo the immune status of an individual, wherein the individual is a trauma patient, a patient with burns, a surgical patient or a patient with sepsis, preferably a patient with septic shock, said method comprising a step of detecting and/or quantifying the expression, in a test biological sample from said individual, also referred to as a test biological sample, of at least a portion of at least one HERV/MaLR sequence selected from the sequences identified in SEQ ID NOs: 1 to 34 or from among the sequences which exhibit at least 99% identity with one of the sequences identified in SEQ ID NOs: 1 to 34, from the following lists:
- List 1 :
| **SEQ ID NO** | **GRCh38 Location** | **Name of the corresponding probeset of the HERV-V3 chip** |
|---|---|---|
| 1 | chr19:54891074-54891496 | 190665001-HERV0376 |
| 2 | chr22:36153696-36154283 | 220247002-HERV0797 |
| 3 | chr17:35505737-35508365 | 170369402HE41env |
| 4 | chr12:112971073-112971451 | 121601801-HERV0492 |
| 5 | chr1:78648318-78648697 | 011052702-MALR1044 |
| 6 | chr1:78623489-78623954 | 011052202-HERV1033 |
| 7 | chr13:42884951-42886257 | 130360601-HERV0808 |
| 8 | chr14:91230494-91230820 | 141107102-MALR1019 |
| 9 | chr2:102363654-102366601 | 021460102-HERV0599uL |
| 10 | chr2:102013616-102013971 | 021456001-MALR1017uL |
| 11 | chr5:14551189-14551685 | 050286701-HERV0513 |
| 12 | chr5:14562791-14563322 | 050287402-MALR1022 |
- List 2 :
| **SEQ ID NO** | **GRCh38 Location** | **Name of the corresponding probeset of the HERV-V3 chip** |
|---|---|---|
| 13 | chr5:132453630-132454148 | 052182701-MALR1129 |
| 14 | chr19:41812466-41813010 | 190478501-MALR1003 |
| 15 | chr1:155637287-155637547 | 011790601ERV9sLU5 |
| 16 | chr5:170290289-170290812 | 052681601-MALR1018 |
| 17 | chr16:50662453-50662912 | 160627301-MALR1014 |
| 18 | chr11:122671887-122672147 | 111686702-HERV0861 |
| 8 | chr14:91230494-91230820 | 141107102-MALR1019 |
| 19 | chr4:15825146-15825565 | 040318302-MALR1134 |
| 20 | chr4:83464568-83464963 | 041529101-MALR1026 |
| 21 | chr14:91222760-91223118 | 141106902-MALR1133 |
- List 3 :
| **SEQ ID NO** | **GRCh38 Location** | **Name of the corresponding probeset of the HERV-V3 chip** |
|---|---|---|
| 1 | chr19:54891074-54891496 | 190665001-HERV0376 |
| 1 | chr19:54891074-54891496 | 190665002-HERV0376 |
| 22 | chr6:18403673-18404108 | 060281701-MALR1043 |
| 23 | chr4:184850413-184850785 | 043166601-MALR1018 |
| 24 | chr10:5856198-5856795 | 100090601-HERV0429 |
| 25 | chr6:107800650-107801138 | 061529601-HERV0492 |
| 26 | chr10:60410534-60411224 | 100871501-MALR1020 |
| 27 | chr17:78345106-78345577 | 170842002-MALR1003 |
- List 4 :
| **SEQ ID NO** | **GRCh38 Location** | **Name of the corresponding probeset of the HERV-V3 chip** |
|---|---|---|
| 28 | chr8:125945973-125951030 | 081921103-HERV0958 |
| 29 | chr3:167401329-167401866 | 032622601MR41sLU5p |
| 30 | chr22:36147793-36148208 | 220246901-HERV0889 |
| 31 | chr6:127790579-127792191 | 061827101-HERV0856 |
| 3 | chr17:35505737-35508365 | 170369402HE41env |
| 32 | chr17:77462942-77463350 | 170828901-HERV0770 |
| 28 | chr8:125945973-125951030 | 081921101-HERV0958 |
| 28 | chr8:125945973-125951030 | 081921102-HERV0958 |
| 33 | chr19:14612123-14612747 | 190148802-MALR1127 |
| 34 | chr12:9038254-9038598 | 120093401-HERV1034 |

2. The method according to claim 1, further comprising the following steps of:
- comparing the expression in the biological test sample, with a reference expression, or with the expression in a reference biological sample,
- then determining the immune status of said individual from said comparison.

3. The method according to any of claims 1 and 2, wherein the expression is detected and/or quantified at the RNA transcript or mRNA level.

4. The method according to claim 3, wherein the expression is detected and/or quantified by a hybridization method, preferably with a hybridization chip, by in situ hybridization or by Northern blot, by an amplification method, preferably by RT-qPCR, or by sequencing, preferably by high throughput sequencing.

5. The method according to any of claims 1 to 4, wherein the immune status is determined as being an immunosuppression status, an immunocompetence status, or an inflammation status.

6. The method according to any of claims 1 to 5, wherein the test biological sample is obtained by sampling made within 10 days following the admission to the medical facility.

7. The method according to any of claims 2 to 6, wherein the reference biological sample is a biological sample taken from a healthy individual, preferably a biological sample taken from the same individual from which the test biological sample was taken but collected before infection or aggression, or a biological sample from an individual of known immune status, preferably with inflammation status, normal immune status, or immunosuppression status.

8. A use of a kit comprising means for amplifying and/or detecting at least one sequence selected from the sequences identified in SEQ Nos: 1 to 34 or from the sequences which have at least 99% identity with one of the identified sequences in SEQ Nos: 1 to 34, **characterized in that** said amplification and/or detection means allow the amplification and/or the detection of at most 100 biomarkers, in total, for determining in vitro or ex vivo the immune status of an individual, wherein the individual is a trauma patient, a patient with burns, a surgical patient or a patient with sepsis, preferably a patient with septic shock

9. The use according to claim 8, wherein:
- the amplification means comprise at least one pair of primers consisting of two amplification primers selected from amplification primers comprising a nucleotide sequence complementary to at least part of a sequence selected from the sequences identified in SEQ Nos: 1 to 34 or from the sequences which exhibit at least 99% identity with one of the sequences identified in SEQ Nos: 1 to 34, and the complementary sequences of these sequences, said at least one pair of amplification primers making it possible to amplify, preferably making it possible to specifically amplify, at least part of a sequence selected from the sequences identified in SEQ Nos: 1 to 34 or from the sequences which exhibit at least 99% of identity with one of the sequences identified in SEQ Nos: 1 to 34, and the sequences complementary to these sequences, and/or
- the detection means comprise at least one hybridization probe whose nucleotide sequence comprises a nucleotide sequence complementary to at least part of a sequence selected from the sequences identified in SEQ Nos: 1 to 34 or from the sequences which exhibit at least 99% identity with one of the sequences identified in SEQ Nos: 1 to 34, and the sequences complementary to these sequences.

10. The use according to claims 8 or 9, **characterized in that** at least one pair of amplification primers is selected from the following pairs of amplification primers:
| **Pair of primers #** | **Primers # (SEQ ID Nos, nucleotide sequence)** |
|---|---|
| 1 | Forward : Primer #1A (SEQ ID No : 35, TGTACAAAACTCAAATGGTCTTC) |
| | Reverse : Primer #1B (SEQ ID No : 36, ATGACCAACTTAGATTTCCTTGA) |
| 2 | Forward : Primer #2A (SEQ ID No : 37, GCCAGAGAGGCATAATGAAGCA) |
| | Reverse : Primer #2B (SEQ ID No : 38, GATTCTAAGCCTCCCCCTCATTT) |
| 3 | Forward : Primer #3A (SEQ ID No : 39, TGGCTCATAGGGATTCCAGACT) |
| | Reverse : Primer #3B (SEQ ID No : 40, AGCAAGTTGTCAAGAGCCAATCT) |
| 4 | Forward : Primer #4A (SEQ ID No : 41, CACTCTAGGAATCTTAGGCA) |
| | Reverse : Primer #4B (SEQ ID No : 42, TGAAACCAATAGTCCAGTG) |
| 5 | Forward : Primer #5A (SEQ ID No : 43, TTCTACTGTTCACTGCTATCCTCC) |
| | Reverse : Primer #5B (SEQ ID No : 44, CCTGTGGCAGCTTTTTGAAGTAA) |
| 6 | Forward : Primer #6A (SEQ ID No : 45, AGAGCAGAAGAAGATGGATACT) |
| | Reverse : Primer #6B (SEQ ID No : 46, CATGAGCTGACATCATCCAAT) |
| 7 | Forward : Primer #7A (SEQ ID No : 47, TCTGTACTGGTTGCCCCAAC) |
| | Reverse : Primer #7B (SEQ ID No : 48, CGTGCCAGGCCTCTAATACTTTT) |
| 8 | Forward : Primer #8A (SEQ ID No : 49, AGGGAAGACCCCAAGATGATG) |
| | Reverse : Primer #8B (SEQ ID No : 50, CATGCAAAGTCCAACGAGAGG) |
| 9 | Forward : Primer #9A (SEQ ID No : 51, GGGTGGCTGCATCCTATGG) |
| | Reverse : Primer #9B (SEQ ID No : 52, CTGGTCAGGAAAAAATTTGCCTTC) |
| 10 | Forward : Primer #10A (SEQ ID No : 53, ACATGACATTGTCTGAACTTTGGG) |
| | Reverse : Primer #10B (SEQ ID No : 54, TAGGACCATGCAGATACTAGTGAC) |
| 11 | Forward : Primer #11A (SEQ ID No : 55, GAACTCCACAAACCTTGA) |
| | Reverse : Primer #11B (SEQ ID No : 56, GCTAGAAGCTTTGGATATCT) |
| 12 | Forward : Primer #12A (SEQ ID No : 57, TGGCTGTTACAACTTTCATG) |
| | Reverse : Primer #12B (SEQ ID No : 58, TCTCCCTATTCTGAGCACA) |

11. The use according to any of claims 8 to 10, wherein at least one hybridization probe is selected from the hybridization probes of sequences SEQ ID No: 59 to 274.

12. The use according to any of claims 8 to 11, further comprising means for amplifying and/or detecting other biomarkers, particularly endogenous biomarkers, such as other HERV/MaLR and/or genes, preferably genes involved in the inflammation and/or the immunity, and/or housekeeping genes, and/or exogenous biomarkers, such as viruses.

13. The use
- of at least one sequence selected from the sequences identified in SEQ Nos: 1 to 34 or from the sequences which have at least 99% identity with one of the sequences identified in SEQ Nos: 1 to 34; and/or
- of at least one pair of amplification primers, consisting of two amplification primers selected from amplification primers comprising a nucleotide sequence complementary to at least part of a sequence selected from the sequences identified in SEQ Nos: 1 to 34 or from the sequences which exhibit at least 99% identity with one of the sequences identified in SEQ Nos: 1 to 34, and the complementary sequences of these sequences, said at least one pair of amplification primers making it possible to amplify, preferably making it possible to specifically amplify, at least part of a sequence selected from the sequences identified in SEQ Nos: 1 to 34 or from the sequences which exhibit at least 99% of identity with one of the sequences identified in SEQ Nos: 1 to 34, and the sequences complementary to these sequences; and/or
- of at least one hybridization probe whose nucleotide sequence comprises a nucleotide sequence complementary to at least part of a sequence selected from the sequences identified in SEQ Nos: 1 to 34 or from the sequences which exhibit at least 99% identity with one of the sequences identified in SEQ Nos: 1 to 34, and the sequences complementary to these sequences; and/or
- for determining in vitro or ex vivo the immune status of an individual, wherein the individual is a trauma patient, a patient with burns, a surgical patient or a patient with sepsis, preferably a patient with septic shock.

14. The use according to claim 13, **characterized in that**:
- at least one pair of amplification primers is selected from the pairs of amplification primers # 1 to 12 according to claim 10; and/or
- at least one hybridization probe is selected from the hybridization probes of sequences SEQ ID No: 59 to 274.
